# EUROPEAN PATENT APPLICATION

(11) **EP 2 857 514 A1**
(43) Date of publication of application: **08.04.2015**
(21) Application number: 13800261.3
(22) Date of filing: 25.04.2013
(51) Int. Cl.: C12N 15/115

(54) **NUCLEIC ACID MOLECULE BINDING TO INFLUENZA VIRUS AND USE THEREFOR**

(30) Priority: 04.06.2012 JP 2012126861
(71) Applicant: NEC Solution Innovators, Ltd., Tokyo 136-8627 (JP)
(72) Inventor: SHIRATORI, Ikuo, Tokyo 136-8627 (JP); AKITOMI, Jou, Tokyo 136-8627 (JP); HORII, Katsunori, Tokyo 136-8627 (JP); FURUICHI, Makio, Tokyo 136-8627 (JP); WAGA, Iwao, Tokyo 136-8627 (JP)
(74) Representative: Betten & Resch
(86) International application number: PCT/JP2013/062256
(87) International publication number: WO 2013/183383

(57) **Abstract**

The present invention provides a nucleic acid molecule applicable to detection of influenza virus. The nucleic acid molecule according to the present invention is a nucleic acid molecule that binds to an influenza virus, including at least one polynucleotide selected from the group consisting of the following polynucleotides (a) to (d):
(a) a polynucleotide that has any of base sequences of SEQ ID NOs: 1 to 30;
(b) a polynucleotide that has a base sequence obtained by deletion, substitution, insertion, and/or addition of one or more bases in any of the base sequences of the polynucleotide (a) and binds to the influenza virus;
(c) a polynucleotide that has a base sequence with an identity of at least 80% to any of the base sequences of the polynucleotide (a) and binds to the influenza virus; and
(d) a polynucleotide that has a base sequence complementary to a polynucleotide hybridizing to any of the base sequences of the polynucleotide (a) under stringent conditions and binds to the influenza virus.

### [Sequence Listing]

TF12067WO sequence list_ST25.txt

SEQUENCE LISTING

<110>  NEC Soft, Ltd

<120>  Nucleic acid molecules which bind to Influenza virus  and use
       thereof.

<130>  1010004889WO01

<150>  JP 2012-126861
<151>  2012-06-04

<160>  53

<170>  PatentIn version 3.5

<210>  1
<211>  73
<212>  DNA
<213>  Artificial Sequence

<220>
<223>  aptamer

<400>  1
ggttagcccg tcccgagata accttggggt ctttgggtgg ggttggtgtg gtcttaacac      60

acggcggctg tag                                                          73


<210>  2
<211>  73
<212>  DNA
<213>  Artificial Sequence

<220>
<223>  aptamer

<400>  2
ggttagcccg tcccgagata acggtctcgg ctcggttctt tcggaaatgt tgcttaacac      60

acggcggctg tag                                                          73


<210>  3
<211>  73
<212>  DNA
<213>  Artificial Sequence

<220>
<223>  aptamer

<400>  3
ggttagcccg tcccgagata acgggtttcg acttgatcgg acttaagcac cgcttaacac      60

acggcggctg tag                                                          73


<210>  4
<211>  74
<212>  DNA
<213>  Artificial Sequence

<220>
<223>  aptamer

<400>  4
ggttagcccg tcccgagata acgggtggtt gggggggcct tttctggttt tgtcttaaca      60

cacggcggct gtag                                                       74


<210>  5
<211>  72
<212>  DNA
<213>  Artificial Sequence

<220>
<223>  aptamer

<400>  5
ggttagcccg tcccgagata actctcggct tggttcgtct gcgaaatgtt acttaacaca      60

cggcggctgt ag                                                         72


<210>  6
<211>  74
<212>  DNA
<213>  Artificial Sequence

<220>
<223>  aptamer

<400>  6
cctgcaccca gtgtcccaca cgtgtatggg ttttttatgg ttaggtaggg gcggcttgac      60

ggagaggagg acgg                                                       74


<210>  7
<211>  75
<212>  DNA
<213>  Artificial Sequence

<220>
<223>  aptamer

<400>  7
cctgcaccca gtgtccccgg cgtttggttg gcgtgaacca tttttaagtt ctgcgtgtga      60

cggagaggag gacgg                                                      75


<210>  8
<211>  74
<212>  DNA
<213>  Artificial Sequence

<220>
<223>  aptamer

<400>  8
cctgcaccca gtgtcccgtc gttggtgttt ttttggcttt tagggtaggt gtggcttgac      60

ggagaggagg acgg                                                       74

<210>  9
<211>  74
<212>  DNA
<213>  Artificial Sequence

<220>
<223>  aptamer

<400>  9
cctgcaccca gtgtccctcg cttctttttt tgggttgggg tgggcgggtt cctgtcggac      60

ggagaggagg acgg                                                        74


<210>  10
<211>  75
<212>  DNA
<213>  Artificial Sequence

<220>
<223>  aptamer

<400>  10
cctgcaccca gtgtccccct ctgtgtcggg cgggccgttc agggttttgg gtgggggtga      60

cggagaggag gacgg                                                       75


<210>  11
<211>  73
<212>  DNA
<213>  Artificial Sequence

<220>
<223>  aptamer

<400>  11
ggttagcccg tcccgagata acctgtgttg ggggtgggag ggtttttggg gtcttaacac      60

acggcggctg tag                                                         73


<210>  12
<211>  73
<212>  DNA
<213>  Artificial Sequence

<220>
<223>  aptamer

<400>  12
ggttagcccg tcccgagata actcggggtg tttgatggtt tggtctggtt ggcttaacac      60

acggcggctg tag                                                         73


<210>  13
<211>  16
<212>  DNA
<213>  Artificial Sequence

<220>
<223>  aptamer

<400>  13
ggtttggtct ggttgg                                                        16


<210>  14
<211>  20
<212>  DNA
<213>  Artificial Sequence

<220>
<223>  aptamer

<400>  14
gtggtttggt ctggttggac                                                    20


<210>  15
<211>  26
<212>  DNA
<213>  Artificial Sequence

<220>
<223>  aptamer

<400>  15
cgtttggttt ggtctggttg gcaacg                                             26


<210>  16
<211>  28
<212>  DNA
<213>  Artificial Sequence

<220>
<223>  aptamer

<400>  16
cgttatggtt tggtctggtt ggctaacg                                           28


<210>  17
<211>  33
<212>  DNA
<213>  Artificial Sequence

<220>
<223>  aptamer

<400>  17
gtgtttgatg gtttggtctg gttggcttaa cac                                     33


<210>  18
<211>  73
<212>  DNA
<213>  Artificial Sequence

<220>
<223>  aptamer

<400>  18
ggttagcccg tcccgagata actcggtgtg ttgggtttgg gttgggttgg gtcttaacac      60

acggcggctg tag                                                          73


<210>  19
<211>  19
<212>  DNA
<213>  Artificial Sequence

<220>
<223>  aptamer

<400>  19
gggtttgggt tgggttggg                                                    19


<210>  20
<211>  87
<212>  DNA
<213>  Artificial Sequence

<220>
<223>  aptamer

<400>  20
atatatatgc ccaccctctc gctgtacggt tgatgcgcgt ttctttctct ttatattgct      60

gggtgccgcc tccaaggtca aaaaaaa                                           87


<210>  21
<211>  60
<212>  DNA
<213>  Artificial Sequence

<220>
<223>  aptamer

<400>  21
gggcaccctc tcgctgtacg gttgatgcac gtttctttct ctttatattg ctgggtgccc      60


<210>  22
<211>  88
<212>  DNA
<213>  Artificial Sequence

<220>
<223>  aptamer

<400>  22
atatatatgc ccaccctctc gctggcggct ctgttctgtt ctcgtctcct tgatttctgt      60

gggcagccgc ctccaaggtc aaaaaaaa                                          88


<210>  23
<211>  62
<212>  DNA
<213>  Artificial Sequence

<220>
<223>  aptamer

<400>  23
ggggcccacc ctctcgctgg cggctctgtt ctgttctcgt ctccttgatt tctgtgggcc      60

cc                                                                      62


<210>  24
<211>  91
<212>  DNA
<213>  Artificial Sequence

<220>
<223>  aptamer

<400>  24
atatatatcc tgcacccagt gtcccgtgtg caattcagtt ggggttattt tgggagggcg      60

ggggttgacg gagaggagga cggaaaaaaa a                                      91


<210>  25
<211>  28
<212>  DNA
<213>  Artificial Sequence

<220>
<223>  aptamer

<400>  25
ttggggttat tttgggaggg cgggggtt                                          28


<210>  26
<211>  76
<212>  DNA
<213>  Artificial Sequence

<220>
<223>  aptamer

<400>  26
cctgcaccca gtgtcccttt cctcgttggg ggtggtggtg ggtttcggtt catgggtggg      60

acggagagga ggacgg                                                       76


<210>  27
<211>  51
<212>  DNA
<213>  Artificial Sequence

<220>
<223>  aptamer

<400>  27
tcctcgttgg gggtggtggt gggtttcggt tcatgggtgg gacggagagg a                51


<210>  28
<211>  74

<212>  DNA
<213>  Artificial Sequence

<220>
<223>  aptamer

<400>  28
cctgcaccca gtgtcccgtg ctccgggggt tgggcgtggt gggtctgtcg ggtttcggac      60

ggagaggagg acgg                                                       74


<210>  29
<211>  74
<212>  DNA
<213>  Artificial Sequence

<220>
<223>  aptamer

<400>  29
cctgcaccca gtgtccctgg gtcggctaat ttggcatttg gggtggtttg gggggggac      60

ggagaggagg acgg                                                       74


<210>  30
<211>  65
<212>  DNA
<213>  Artificial Sequence

<220>
<223>  aptamer

<400>  30
cctgcaccca gtgtccctgt gtgctggtgt ggggtggttt ggggggggga cggagaggag      60

gacgg                                                                 65


<210>  31
<211>  39
<212>  DNA
<213>  Artificial Sequence

<220>
<223>  aptamer


<220>
<221>  misc_feature
<222>  (20)..(20)
<223>  n represents one ore more any bases

<400>  31
gggtttgggt tgggttgggn gggtttgggt tgggttggg                            39


<210>  32
<211>  47
<212>  DNA
<213>  Artificial Sequence

<220>
<223>  aptamer

<400>  32
gggtttgggt tgggttgggt ttttttttgg gtttgggttg ggttggg                    47


<210>  33
<211>  43
<212>  DNA
<213>  Artificial Sequence

<220>
<223>  aptamer

<400>  33
gggtttgggt tgggttgggt ttttgggttt gggttgggtt ggg                         43


<210>  34
<211>  44
<212>  DNA
<213>  Artificial Sequence

<220>
<223>  aptamer


<220>
<221>  misc_feature
<222>  (23)..(23)
<223>  n represents random 30-mer sequence.

<400>  34
ggttagcccg tcccgagata acncttaaca cacggcggct gtag                       44


<210>  35
<211>  30
<212>  DNA
<213>  Artificial Sequence

<220>
<223>  aptamer


<220>
<221>  misc_feature
<222>  (13)..(13)
<223>  n represents random 40-mer sequence.

<400>  35
acccagtgtc ccngacggag aggaggacgg                                       30


<210>  36
<211>  337
<212>  PRT
<213>  Influenza virus/A/Anhui/1/2005

<400>  36

Met Glu Lys Ile Val Leu Leu Leu Ala Ile Val Ser Leu Val Lys Ser

1               5                   10                  15

Asp Gln Ile Cys Ile Gly Tyr His Ala Asn Asn Ser Thr Glu Gln Val
            20                  25                  30

Asp Thr Ile Met Glu Lys Asn Val Thr Val Thr His Ala Gln Asp Ile
        35                  40                  45

Leu Glu Lys Thr His Asn Gly Lys Leu Cys Asp Leu Asp Gly Val Lys
    50                  55                  60

Pro Leu Ile Leu Arg Asp Cys Ser Val Ala Gly Trp Leu Leu Gly Asn
65                  70                  75                  80

Pro Met Cys Asp Glu Phe Ile Asn Val Pro Glu Trp Ser Tyr Ile Val
                85                  90                  95

Glu Lys Ala Asn Pro Ala Asn Asp Leu Cys Tyr Pro Gly Asn Phe Asn
            100                 105                 110

Asp Tyr Glu Glu Leu Lys His Leu Leu Ser Arg Ile Asn His Phe Glu
        115                 120                 125

Lys Ile Gln Ile Ile Pro Lys Ser Ser Trp Ser Asp His Glu Ala Ser
    130                 135                 140

Ser Gly Val Ser Ser Ala Cys Pro Tyr Gln Gly Thr Pro Ser Phe Phe
145                 150                 155                 160

Arg Asn Val Val Trp Leu Ile Lys Lys Asn Asn Thr Tyr Pro Thr Ile
                165                 170                 175

Lys Arg Ser Tyr Asn Asn Thr Asn Gln Glu Asp Leu Leu Ile Leu Trp
            180                 185                 190

Gly Ile His His Ser Asn Asp Ala Ala Glu Gln Thr Lys Leu Tyr Gln
        195                 200                 205

Asn Pro Thr Thr Tyr Ile Ser Val Gly Thr Ser Thr Leu Asn Gln Arg
    210                 215                 220

Leu Val Pro Lys Ile Ala Thr Arg Ser Lys Val Asn Gly Gln Ser Gly
225                 230                 235                 240

Arg Met Asp Phe Phe Trp Thr Ile Leu Lys Pro Asn Asp Ala Ile Asn
                245                 250                 255

Phe Glu Ser Asn Gly Asn Phe Ile Ala Pro Glu Tyr Ala Tyr Lys Ile
            260                 265                 270

Val Lys Lys Gly Asp Ser Ala Ile Val Lys Ser Glu Val Glu Tyr Gly
        275                 280                 285

Asn Cys Asn Thr Lys Cys Gln Thr Pro Ile Gly Ala Ile Asn Ser Ser
    290                 295                 300

Met Pro Phe His Asn Ile His Pro Leu Thr Ile Gly Glu Cys Pro Lys
305                 310                 315                 320

Tyr Val Lys Ser Asn Lys Leu Val Leu Ala Thr Gly Leu Arg Asn Ser
                325                 330                 335

Pro

<210> 37
<211> 338
<212> PRT
<213> Influenza virus/A/goose-Guiyang/337/2006

<400> 37

Met Glu Lys Ile Val Leu Leu Leu Ala Ile Ile Ser Leu Val Lys Ser
1               5                   10                  15

Asp Gln Ile Cys Ile Gly Tyr His Ala Asn Asn Ser Thr Val Gln Val
            20                  25                  30

Asp Thr Ile Met Glu Lys Asn Val Thr Val Thr His Ala Gln Asp Ile
        35                  40                  45

Leu Glu Lys Thr His Asn Gly Lys Leu Cys Ser Leu Asp Gly Val Lys
    50                  55                  60

Pro Leu Ile Leu Arg Asp Cys Ser Val Ala Gly Trp Leu Leu Gly Asn
65                  70                  75                  80

Pro Met Cys Asp Glu Phe Ile Asn Val Pro Glu Trp Ser Tyr Ile Val
                85                  90                  95

Glu Lys Ala Ser Pro Ala Asn Asp Leu Cys Tyr Pro Gly Asp Phe Asn
            100                 105                 110

Asp Tyr Glu Glu Leu Lys His Leu Leu Ser Arg Ile Asn His Phe Glu
        115                 120                 125

Lys Ile Gln Ile Ile Pro Lys Ser Ser Trp Pro Asn His Glu Ala Ser
    130                 135                 140

Leu Gly Val Ser Ser Ala Cys Pro Tyr Gln Gly Glu Ser Ser Phe Phe
145                 150                 155                 160

Arg Asn Val Val Trp Leu Ile Lys Lys Asn Ser Ser Tyr Pro Thr Ile
                165                 170                 175

Lys Arg Ser Tyr Asn Asn Thr Asn Gln Glu Asp Leu Leu Val Leu Trp
            180                 185                 190

Gly Ile His His Pro Asn Asp Ala Ala Glu Gln Ile Lys Leu Tyr Gln
        195                 200                 205

Asn Pro Asn Thr Tyr Ile Ser Val Gly Thr Ser Thr Leu Asn Gln Arg
    210                 215                 220

Leu Val Pro Thr Ile Ala Thr Arg Ser Lys Val Asn Gly Gln Ser Gly
225                 230                 235                 240

Arg Met Glu Phe Phe Trp Thr Ile Leu Lys Pro Asn Asp Ala Ile Asn
                245                 250                 255

Phe Glu Ser Asn Gly Asn Phe Ile Ala Pro Glu Tyr Ala Tyr Lys Ile
            260                 265                 270

Val Lys Lys Gly Asp Ser Ala Ile Met Lys Ser Glu Leu Glu Tyr Gly
        275                 280                 285

Asn Cys Asn Thr Lys Cys Gln Thr Pro Met Ile Gly Ala Ile Asn Ser
    290                 295                 300

Ser Met Pro Phe His Asn Ile His Pro Leu Thr Ile Gly Glu Cys Pro
305                 310                 315                 320

Lys Tyr Val Lys Ser Asn Arg Leu Val Leu Ala Thr Gly Leu Arg Asn
                325                 330                 335

Thr Leu

<210>  38
<211>  523
<212>  PRT
<213>  Influenza virus/A/Anhui/1/2005

<400>  38

Met Glu Lys Ile Val Leu Leu Leu Ala Ile Val Ser Leu Val Lys Ser
1               5                   10                  15

Asp Gln Ile Cys Ile Gly Tyr His Ala Asn Asn Ser Thr Glu Gln Val
            20                  25                  30

Asp Thr Ile Met Glu Lys Asn Val Thr Val Thr His Ala Gln Asp Ile
        35                  40                  45

Leu Glu Lys Thr His Asn Gly Lys Leu Cys Asp Leu Asp Gly Val Lys
    50                  55                  60

Pro Leu Ile Leu Arg Asp Cys Ser Val Ala Gly Trp Leu Leu Gly Asn
65                  70                  75                  80

Pro Met Cys Asp Glu Phe Ile Asn Val Pro Glu Trp Ser Tyr Ile Val
                85                  90                  95

Glu Lys Ala Asn Pro Ala Asn Asp Leu Cys Tyr Pro Gly Asn Phe Asn
            100                 105                 110

Asp Tyr Glu Glu Leu Lys His Leu Leu Ser Arg Ile Asn His Phe Glu
        115                 120                 125

Lys Ile Gln Ile Ile Pro Lys Ser Ser Trp Ser Asp His Glu Ala Ser
    130                 135                 140

Ser Gly Val Ser Ser Ala Cys Pro Tyr Gln Gly Thr Pro Ser Phe Phe
145                 150                 155                 160

Arg Asn Val Val Trp Leu Ile Lys Lys Asn Asn Thr Tyr Pro Thr Ile
                165                 170                 175

Lys Arg Ser Tyr Asn Asn Thr Asn Gln Glu Asp Leu Leu Ile Leu Trp
            180                 185                 190

Gly Ile His His Ser Asn Asp Ala Ala Glu Gln Thr Lys Leu Tyr Gln
        195                 200                 205

Asn Pro Thr Thr Tyr Ile Ser Val Gly Thr Ser Thr Leu Asn Gln Arg
    210                 215                 220

Leu Val Pro Lys Ile Ala Thr Arg Ser Lys Val Asn Gly Gln Ser Gly
225                 230                 235                 240

Arg Met Asp Phe Phe Trp Thr Ile Leu Lys Pro Asn Asp Ala Ile Asn
                245                 250                 255

Phe Glu Ser Asn Gly Asn Phe Ile Ala Pro Glu Tyr Ala Tyr Lys Ile
            260                 265                 270

Val Lys Lys Gly Asp Ser Ala Ile Val Lys Ser Glu Val Glu Tyr Gly
        275                 280                 285

Asn Cys Asn Thr Lys Cys Gln Thr Pro Ile Gly Ala Ile Asn Ser Ser
    290                 295                 300

Met Pro Phe His Asn Ile His Pro Leu Thr Ile Gly Glu Cys Pro Lys
305                 310                 315                 320

Tyr Val Lys Ser Asn Lys Leu Val Leu Ala Thr Gly Leu Arg Asn Ser
                325                 330                 335

Pro Arg Gly Leu Phe Gly Ala Ile Ala Gly Phe Ile Glu Gly Gly Trp
            340                 345                 350

Gln Gly Met Val Asp Gly Trp Tyr Gly Tyr His His Ser Asn Glu Gln
        355                 360                 365

Gly Ser Gly Tyr Ala Ala Asp Lys Glu Ser Thr Gln Lys Ala Ile Asp
    370                 375                 380

Gly Val Thr Asn Lys Val Asn Ser Ile Ile Asp Lys Met Asn Thr Gln
385                 390                 395                 400

Phe Glu Ala Val Gly Arg Glu Phe Asn Asn Leu Glu Arg Arg Ile Glu
                405                 410                 415

Asn Leu Asn Lys Lys Met Glu Asp Gly Phe Leu Asp Val Trp Thr Tyr
            420                 425                 430

Asn Ala Glu Leu Leu Val Leu Met Glu Asn Glu Arg Thr Leu Asp Phe
        435                 440                 445

His Asp Ser Asn Val Lys Asn Leu Tyr Asp Lys Val Arg Leu Gln Leu
    450                 455                 460

Arg Asp Asn Ala Lys Glu Leu Gly Asn Gly Cys Phe Glu Phe Tyr His
465                 470                 475                 480

Lys Cys Asp Asn Glu Cys Met Glu Ser Val Arg Asn Gly Thr Tyr Asp
                485                 490                 495

Tyr Pro Gln Tyr Ser Glu Glu Ala Arg Leu Lys Arg Glu Glu Ile Ser
            500                 505                 510

Gly Val Lys Leu Glu Ser Ile Gly Thr Tyr Gln
        515                 520

<210>  39
<211>  33
<212>  DNA
<213>  Artificial Sequence

<220>
<223>  aptamer

<400>  39
cggcgtttgg ttggcgtgaa ccatttttaa gtt                                    33

<210>  40
<211>  68
<212>  DNA
<213>  Artificial Sequence

<220>
<223>  aptamer

<400>  40
aattaaccct cactaaaggg ctgagtctca aaaccgcaat acactggttg tatggtcgaa       60

taagttaa                                                                68

<210>  41
<211>  344
<212>  PRT
<213>  Influenza virus/A/california/04/2009

<400>  41

Met Lys Ala Ile Leu Val Val Leu Leu Tyr Thr Phe Ala Thr Ala Asn
1               5                   10                  15

Ala Asp Thr Leu Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asp Lys His Asn Gly Lys Leu Cys Lys Leu Arg Gly Val
    50                  55                  60

Ala Pro Leu His Leu Gly Lys Cys Asn Ile Ala Gly Trp Ile Leu Gly
65                  70                  75                  80

Asn Pro Glu Cys Glu Ser Leu Ser Thr Ala Ser Ser Trp Ser Tyr Ile
                85                  90                  95

Val Glu Thr Pro Ser Ser Asp Asn Gly Thr Cys Tyr Pro Gly Asp Phe
        100                 105                 110

Ile Asp Tyr Glu Glu Leu Arg Glu Gln Leu Ser Ser Val Ser Ser Phe
        115                 120                 125

Glu Arg Phe Glu Ile Phe Pro Lys Thr Ser Ser Trp Pro Asn His Asp
    130                 135                 140

Ser Asn Lys Gly Val Thr Ala Ala Cys Pro His Ala Gly Ala Lys Ser
145                 150                 155                 160

Phe Tyr Lys Asn Leu Ile Trp Leu Val Lys Lys Gly Asn Ser Tyr Pro
                165                 170                 175

Lys Leu Ser Lys Ser Tyr Ile Asn Asp Lys Gly Lys Glu Val Leu Val
            180                 185                 190

Leu Trp Gly Ile His His Pro Ser Thr Ser Ala Asp Gln Gln Ser Leu
        195                 200                 205

Tyr Gln Asn Ala Asp Thr Tyr Val Phe Val Gly Ser Ser Arg Tyr Ser
    210                 215                 220

Lys Lys Phe Lys Pro Glu Ile Ala Ile Arg Pro Lys Val Arg Asp Gln
225                 230                 235                 240

Glu Gly Arg Met Asn Tyr Tyr Trp Thr Leu Val Glu Pro Gly Asp Lys
                245                 250                 255

Ile Thr Phe Glu Ala Thr Gly Asn Leu Val Val Pro Arg Tyr Ala Phe
            260                 265                 270

Ala Met Glu Arg Asn Ala Gly Ser Gly Ile Ile Ile Ser Asp Thr Pro
        275                 280                 285

Val His Asp Cys Asn Thr Thr Cys Gln Thr Pro Lys Gly Ala Ile Asn
    290                 295                 300

Thr Ser Leu Pro Phe Gln Asn Ile His Pro Ile Thr Ile Gly Lys Cys
305                 310                 315                 320

Pro Lys Tyr Val Lys Ser Thr Lys Leu Arg Leu Ala Thr Gly Leu Arg
                325                 330                 335

Asn Ile Pro Ser Ile Gln Ser Arg
            340

<210>  42
<211>  343
<212>  PRT
<213>  Influenza virus/A/Brisbane/59/2007

<400>  42

Met Lys Val Lys Leu Leu Val Leu Leu Cys Thr Phe Thr Ala Thr Tyr
1               5                   10                  15

Ala Asp Thr Ile Cys Ile Gly Tyr His Ala Asn Asn Ser Thr Asp Thr
            20                  25                  30

Val Asp Thr Val Leu Glu Lys Asn Val Thr Val Thr His Ser Val Asn
        35                  40                  45

Leu Leu Glu Asn Ser His Asn Gly Lys Leu Cys Leu Leu Lys Gly Ile
    50                  55                  60

Ala Pro Leu Gln Leu Gly Asn Cys Ser Val Ala Gly Trp Ile Leu Gly
65                  70                  75                  80

Asn Pro Glu Cys Glu Leu Leu Ile Ser Lys Glu Ser Trp Ser Tyr Ile
                85                  90                  95

Val Glu Lys Pro Asn Pro Glu Asn Gly Thr Cys Tyr Pro Gly His Phe
            100                 105                 110

Ala Asp Tyr Glu Glu Leu Arg Glu Gln Leu Ser Ser Val Ser Ser Phe
        115                 120                 125

Glu Arg Phe Glu Ile Phe Pro Lys Glu Ser Ser Trp Pro Asn His Thr
    130                 135                 140

Val Thr Gly Val Ser Ala Ser Cys Ser His Asn Gly Glu Ser Ser Phe
145                 150                 155                 160

Tyr Arg Asn Leu Leu Trp Leu Thr Gly Lys Asn Gly Leu Tyr Pro Asn
                165                 170                 175

Leu Ser Lys Ser Tyr Ala Asn Asn Lys Glu Lys Glu Val Leu Val Leu
            180                 185                 190

Trp Gly Val His His Pro Pro Asn Ile Gly Asp Gln Lys Ala Leu Tyr
        195                 200                 205

His Thr Glu Asn Ala Tyr Val Ser Val Val Ser Ser His Tyr Ser Arg
    210                 215                 220

Lys Phe Thr Pro Glu Ile Ala Lys Arg Pro Lys Val Arg Asp Gln Glu
225                 230                 235                 240

Gly Arg Ile Asn Tyr Tyr Trp Thr Leu Leu Glu Pro Gly Asp Thr Ile
                245                 250                 255

Ile Phe Glu Ala Asn Gly Asn Leu Ile Ala Pro Arg Tyr Ala Phe Ala
            260                 265                 270

Leu Ser Arg Gly Phe Gly Ser Gly Ile Ile Asn Ser Asn Ala Pro Met
        275                 280                 285

Asp Lys Cys Asp Ala Lys Cys Gln Thr Pro Gln Gly Ala Ile Asn Ser
    290                 295                 300

Ser Leu Pro Phe Gln Asn Val His Pro Val Thr Ile Gly Glu Cys Pro
305                 310                 315                 320

Lys Tyr Val Arg Ser Ala Lys Leu Arg Met Val Thr Gly Leu Arg Asn
                325                 330                 335

Ile Pro Ser Ile Gln Ser Arg
            340

<210>  43
<211>  345
<212>  PRT
<213>  Influenza virus/A/Aichi/2/1968

<400>  43

Met Lys Thr Ile Ile Ala Leu Ser Tyr Ile Phe Cys Leu Ala Leu Gly
1               5                   10                  15

Gln Asp Leu Pro Gly Asn Asp Asn Ser Thr Ala Thr Leu Cys Leu Gly
            20                  25                  30

His His Ala Val Pro Asn Gly Thr Leu Val Lys Thr Ile Thr Asp Asp
        35                  40                  45

Gln Ile Glu Val Thr Asn Ala Thr Glu Leu Val Gln Ser Ser Ser Thr
    50                  55                  60

Gly Lys Ile Cys Asn Asn Pro His Arg Ile Leu Asp Gly Ile Asp Cys
65                  70                  75                  80

Thr Leu Ile Asp Ala Leu Leu Gly Asp Pro His Cys Asp Val Phe Gln
                85                  90                  95

Asn Glu Thr Trp Asp Leu Phe Val Glu Arg Ser Lys Ala Phe Ser Asn
            100                 105                 110

Cys Tyr Pro Tyr Asp Val Pro Asp Tyr Ala Ser Leu Arg Ser Leu Val
        115                 120                 125

Ala Ser Ser Gly Thr Leu Glu Phe Ile Thr Glu Gly Phe Thr Trp Thr
    130                 135                 140

Gly Val Thr Gln Asn Gly Gly Ser Asn Ala Cys Lys Arg Gly Pro Gly
145                 150                 155                 160

Ser Gly Phe Phe Ser Arg Leu Asn Trp Leu Thr Lys Ser Gly Ser Thr
                165                 170                 175

Tyr Pro Val Leu Asn Val Thr Met Pro Asn Asn Asp Asn Phe Asp Lys
            180                 185                 190

Leu Tyr Ile Trp Gly Ile His His Pro Ser Thr Asn Gln Glu Gln Thr
        195                 200                 205

Ser Leu Tyr Val Gln Ala Ser Gly Arg Val Thr Val Ser Thr Arg Arg
    210                 215                 220

Ser Gln Gln Thr Ile Ile Pro Asn Ile Gly Ser Arg Pro Trp Val Arg
225                 230                 235                 240

Gly Leu Ser Ser Arg Ile Ser Ile Tyr Trp Thr Ile Val Lys Pro Gly
                245                 250                 255

Asp Val Leu Val Ile Asn Ser Asn Gly Asn Leu Ile Ala Pro Arg Gly
            260                 265                 270

Tyr Phe Lys Met Arg Thr Gly Lys Ser Ser Ile Met Arg Ser Asp Ala
        275                 280                 285

Pro Ile Asp Thr Cys Ile Ser Glu Cys Ile Thr Pro Asn Gly Ser Ile
    290                 295                 300

Pro Asn Asp Lys Pro Phe Gln Asn Val Asn Lys Ile Thr Tyr Gly Ala
305                 310                 315                 320

Cys Pro Lys Tyr Val Lys Gln Asn Thr Leu Lys Leu Ala Thr Gly Met
                325                 330                 335

Arg Asn Val Pro Glu Lys Gln Thr Arg
            340                 345

<210>  44
<211>  48
<212>  DNA
<213>  Artificial Sequence

<220>
<223>  antisense

<400>  44
cccaaaccca acccaaccca aaaacccaaa cccaacccaa ccctttttt                48

<210>  45
<211>  67
<212>  DNA
<213>  Artificial Sequence

<220>
<223>  aptamer

<220>
<221>  misc_feature
<222>  (34)..(34)
<223>  n represents one or more any bases.

<400>  45
gtgtttgatg gtttggtctg gttggcttaa cacngtgttt gatggtttgg tctggttggc      60

ttaacac                                                                67

<210>  46
<211>  47
<212>  DNA
<213>  Artificial Sequence

<220>
<223>  aptamer

<400>  46
gggtttgggt tgggttggga tatatatagg gtttgggttg ggttggg                    47

<210>  47
<211>  58
<212>  DNA
<213>  Artificial Sequence

<220>
<223>  aptamer

<400>  47
gggtttgggt tgggttgggg attggaatta aggcgtgtgg ggtttgggtt gggttggg        58

<210>  48
<211>  75
<212>  DNA
<213>  Artificial Sequence

<220>
<223>  aptamer

<400>  48
gtgtttgatg gtttggtctg gttggcttaa cacttttttt ttgtgtttga tggtttggtc      60

tggttggctt aacac                                                      75


<210>  49
<211>  71
<212>  DNA
<213>  Artificial Sequence

<220>
<223>  aptamer

<400>  49
gtgtttgatg gtttggtctg gttggcttaa cactttttgt gtttgatggt ttggtctggt      60

tggcttaaca c                                                          71


<210>  50
<211>  59
<212>  DNA
<213>  Artificial Sequence

<220>
<223>  aptamer


<220>
<221>  misc_feature
<222>  (20)..(20)
<223>  n represents one or more any bases.

<220>
<221>  misc_feature
<222>  (40)..(40)
<223>  n represents one or more any bases.

<400>  50
gggtttgggt tgggttgggn gggtttgggt tgggttgggn gggtttgggt tgggttggg      59


<210>  51
<211>  75
<212>  DNA
<213>  Artificial Sequence

<220>
<223>  aptamer

<400>  51
gggtttgggt tgggttgggt ttttttttgg gtttgggttg ggttgggttt ttttttgggt      60

ttgggttggg ttggg                                                      75


<210>  52
<211>  79
<212>  DNA

<213>  Artificial Sequence

<220>
<223>  aptamer

<400>  52
gaattcagtc ggacagcggg gttcccatgc ggatgttata aagcagtcgc ttataaggga        60

tggacgaata tcgtctccc                                                     79


<210>  53
<211>  79
<212>  DNA
<213>  Artificial Sequence

<220>
<223>  aptamer

<400>  53
gaattcagtc ggacagcggg ggggaattct agcagtacac ctcgagaatt atagccagga        60

tggacgaata tcgtctccc                                                     79

## Description

### Technical Field

The present invention relates to a nucleic acid molecule that binds to influenza viruses and use thereof.

### Background Art

Spread of infection with seasonal influenza viruses and novel influenza viruses have been seen in recent years, and influenza virus detection is becoming more and more important.

In influenza virus detection, a method utilizing gene amplification or a method using an antibody has been used, for example. The former is a method in which nucleic acids in a sample are subjected to PCR or the like so as to amplify a base sequence unique to an influenza virus and the presence or absence of infection with the influenza virus is determined depending on whether or not the amplification has occurred. However, in order to carry out gene amplification, it is necessary to, for example, pretreat a sample collected from a human body, which takes time and effort. Also, there is a problem of false positive errors due to the amplification of similar sequences etc.

On the other hand, an antibody has a problem in that preparation thereof itself is a very complicated operation and requires high cost, for example. The antibody also has a problem concerning the detection accuracy, because of the possibility of non-specific binding to antigens other than a target antigen. On account of these problems, in recent years, a nucleic acid molecule that specifically binds to an antigen is attracting attention as a substitute for the antibody (Non-Patent Documents 1 and 2). However, previously reported nucleic acid molecules that bind to influenza viruses have insufficient binding ability. Hence, a novel nucleic acid molecule is demanded.

### Citation List

### Non-Patent Document(s)

[Non-Patent Document 1] Jeon et al., J. Biol Chem. 2004 279 (46), 48410-48419
[Non-Patent Document 2] Cheng et al., Biochem Biophys Res Commun. 2008 366 (3), 670-674

### Brief Summary of the Invention

### Problems to be Solved by the Invention

With the foregoing in mind, it is an object of the present invention to provide a nucleic acid molecule that can be used in detection and the like of influenza viruses. Means for Solving Problems

The present invention provides a nucleic acid molecule that binds to an influenza virus, including at least one polynucleotide selected from the group consisting of the following polynucleotides (a) to (d):
(a) a polynucleotide that has any of base sequences of SEQ ID NOs: 1 to 30;
(b) a polynucleotide that has a base sequence obtained by deletion, substitution, insertion, and/or addition of one or more bases in any of the base sequences of the polynucleotide (a) and binds to the influenza virus;
(c) a polynucleotide that has a base sequence with an identity of at least 80% to any of the base sequences of the polynucleotide (a) and binds to the influenza virus; and
(d) a polynucleotide that has a base sequence complementary to a polynucleotide hybridizing to any of the base sequences of the polynucleotide (a) under stringent conditions and binds to the influenza virus.

The present invention also provides a binding agent that binds to an influenza virus, containing the nucleic acid molecule according to the present invention.

### Effects of the Invention

The nucleic acid molecule according to the present invention can bind to influenza viruses, and thus can detect influenza viruses, for example. Therefore, the nucleic acid molecule according to the present invention can be a very useful tool for the detection of influenza viruses in the fields of clinical practice, animal husbandry, and the like, for example. Brief Description of Drawings
[FIG. 1] FIG 1 is a schematic view showing predicted secondary structures of polynucleotides in the present invention.
[FIG. 2] FIG. 2 is a schematic view showing predicted secondary structures of other polynucleotides in the present invention.
[FIG. 3] FIG. 3 is a schematic view showing predicted secondary structures of still other polynucleotides in the present invention.
[FIG. 4] FIG. 4 is a graph showing the binding ability of nucleic acid molecules to target proteins in Example A1 of the present invention.
[FIG 5] FIG. 5 is a graph showing the binding ability of the nucleic acid molecules to antibodies in Example A1 of the present invention.
[FIG. 6] FIG. 6 is a graph showing the binding ability of nucleic acid molecules to target proteins in Example A4 of the present invention.
[FIG. 7] FIG 7 is a graph showing the binding ability of the nucleic acid molecules to antibodies in Example A4 of the present invention.
[FIG. 8] FIG. 8 is a graph showing the binding ability of each nucleic acid molecule, each HA, and each antibody in Example A6 of the present invention.
[FIG 9] FIG. 9 is a graph showing the binding ability of nucleic acid molecules to target proteins in Example B1 of the present invention.
[FIG. 10] FIG. 10 is a graph showing the binding ability of nucleic acid molecules to target proteins in Example B2 of the present invention.
[FIG. 11] FIG. 11 is a graph showing the binding ability of nucleic acid molecules to a target protein in Example B3 of the present invention.
[FIG 12] FIG. 12 is a graph showing the binding ability of nucleic acid molecules to a target protein in Example B4 of the present invention.
[FIG. 13] FIG 13 is a graph showing the binding ability of nucleic acid molecules to a target protein in Example B5 of the present invention.
[FIG. 14] FIG 14 is a graph showing the binding ability of nucleic acid molecules to target proteins in Example B5 of the present invention.
[FIG. 15] FIG. 15 is a graph showing the binding ability of a nucleic acid molecule to a target protein in Example B6 of the present invention.
[FIG. 16] FIG 16 is a graph showing the binding ability of nucleic acid molecules to target proteins in Example B7 of the present invention.
[FIG. 17] FIG. 17 is a graph showing the binding ability of nucleic acid molecules to a target protein in Example C1 of the present invention.
[FIG. 18] FIG. 18 is a graph showing the binding ability of nucleic acid molecules to target proteins in Example C2 of the present invention.
[FIG. 19] FIG 19 is a schematic view showing predicted secondary structures of still other polynucleotides in the present invention.
[FIG 20] FIG. 20 is a schematic view showing predicted secondary structures of still other polynucleotides in the present invention.
[FIG. 21] FIG. 21 is a schematic view showing predicted secondary structures of still other polynucleotides in the present invention.
[FIG. 22] FIG. 22 is a schematic view showing predicted secondary structures of still other polynucleotides in the present invention.
[FIG. 23] FIG. 23 is a schematic view showing predicted secondary structures of still other polynucleotides in the present invention.
[FIG. 24] FIG 24 is a schematic view showing predicted secondary structures of still other polynucleotides in the present invention.
[FIG. 25] FIG. 25 is a graph showing the binding ability of nucleic acid molecules to influenza viruses in Example D1 of the present invention.
[FIG. 26] FIG. 26 is a graph showing the binding ability of nucleic acid molecules to influenza viruses in Example D1 of the present invention.
[FIG. 27] FIG. 27 is a graph showing the binding ability of nucleic acid molecules to influenza viruses in Example D2 of the present invention.

Mode for Carrying out the Invention

As described above, the nucleic acid molecule of the present invention is a nucleic acid molecule that binds to an influenza virus, including at least one polynucleotide selected from the group consisting of the following polynucleotides (a) to (d):
(a) a polynucleotide that has any of base sequences of SEQ ID NOs: 1 to 30;
(b) a polynucleotide that has a base sequence obtained by deletion, substitution, insertion, and/or addition of one or more bases in any of the base sequences of the polynucleotide (a) and binds to the influenza virus;
(c) a polynucleotide that has a base sequence with an identity of at least 80% to any of the base sequences of the polynucleotide (a) and binds to the influenza virus; and
(d) a polynucleotide that has a base sequence complementary to a polynucleotide hybridizing to any of the base sequences of the polynucleotide (a) under stringent conditions and binds to the influenza virus.

In the present invention, "binding to an influenza virus (and grammatical variations thereof)" also is referred to as, for example, "having binding ability to an influenza virus" or "having binding activity to an influenza virus". The binding between the nucleic acid molecule of the present invention and an influenza virus can be determined by, for example, surface plasmon resonance (SPR) analysis and the like. The analysis can be carried out using a ProteON (trade name, BioRad), for example.

The type of influenza virus to which the present invention is applicable is by no means limited. Examples of the influenza virus include influenza A virus, influenza B virus, and influenza C virus. Influenza A virus has 144 subtypes resulting from the combinations of 16 types (H1 to H16) of hemagglutinin (HA) and 9 types (N1 to N9) of neuraminidase (NA), and the subtype to which the present invention is applicable is not particularly limited. In particular, it maybe H5N1, H1N1, or H3N2, for example. More specifically, examples of H5N1 include A/Anhui/1/2005 and A/goose_Guiyang/337/2006, A/Japanese white eye/Hong Kong/1038/2006; examples of H1N1 include A/California/04/2009, A/Brisbane/59/2007, and A/Georgia/20/2006; and examples of H3N2 include A/Aichi/2/1968, A/Wisconsin/67/2005, A/Brisbane/59/2007, and A/Moscow/10/1999. In influenza B virus, only the combination of 1 type of HA and 1 type of NA is present. Examples of the influenza B virus include B/Florida/4/2006 and B/Malaysia/2506/2004.

The nucleic acid molecule of the present invention can bind to influenza virus-derived hemagglutinin (HA), for example. More specifically, the nucleic acid molecule of the present invention can bind to the HA, HA1 as a subunit of the HA, and a complex of HA1 and H2, for example. In the present invention, "binding to influenza virus-derived HA (and grammatical variations thereof)" also is referred to as, for example, "having binding ability to the HA" or "having binding activity to the HA". The binding between the nucleic acid molecule of the present invention and the HA can be determined by the same analyses as those described above. The influenza virus from which the HA is derived is not particularly limited, and examples thereof includes the above-described influenza viruses. In the present invention, hereinafter, "binding to an influenza virus (and grammatical variations thereof)" should be interpreted as interchangeable with "binding to influenza virus-derived HA", for example.

In the nucleic acid molecule of the present invention, the building blocks of the polynucleotides (a) to (d) are, for example, nucleotide residues, examples of which include deoxyribonucleotide residues and ribonucleotide residues. The polynucleotide is, for example, DNA consisting of deoxyribonucleotide residues or DNA including a ribonucleotide residue(s), which both may further include a non-nucleotide residue(s), for example, as described below. The nucleic acid according to the present invention hereinafter also is referred to as "DNA aptamer", for example.

The nucleic acid molecule according to the present invention may consist of any of the polynucleotides (a) to (d) or may include any of the polynucleotides (a) to (d), for example. In the latter case, the nucleic acid molecule of the present invention may include, for example, two or more polynucleotides selected from the polynucleotides (a) to (d), as described below. The two or more polynucleotides may have either the same sequence or different sequences. Also, in the latter case, the nucleic acid molecule of the present invention further may include a linker(s) and/or an additional sequence(s), for example.

The polynucleotide (a) has any of base sequences of SEQ ID NOs: 1 to 30. RHA0023 (SEQ ID NO: 1) 
GGTTAGCCCGTCCCGAGATAACCTTGGGGTCTTTGGGTGGGGTTGGTGTGGTCTTA
ACACACGGCGGCTGTAG
 
RHA0030 (SEQ ID NO: 2)
GGTTAGCCCGTCCCGAGATAACGGTCTCGGCTCGGTTCTTTCGGAAATGTTGCTTA
ACACACGGCGGCTGTAG
 
RHA0013 (SEQ ID NO: 3)
GGTTAGCCCGTCCCGAGATAACGGGTTTCGACTTGATCGGACTTAAGCACCGCTTA
ACACACGGCGGCTGTAG
 
RHA0009 (SEQ ID NO: 4)
GGTTAGCCCGTCCCGAGATAACGGGTGGTTGGGGGGCCTTTTCTGGTTTTGTCTT
AACACACGGCGGCTGTAG
 
RHA0058 (SEQ ID NO: 5)
GGTTAGCCCGTCCCGAGATAACTCTCGGCTTGGTTCGTCTGCGAAATGTTACTTAA
CACACGGCGGCTGTAG
 
RHA0099 (SEQ ID NO: 6)
CCTGCACCCAGTGTCCCACACGTGTATGGGTTTTTATGGTTAGGTAGGGGCGGCT
TGACGGAGAGGAGGACGG
 
RHA0110 (SEQ ID NO: 7)
CCTGCACCCAGTGTCCCCGGCGTTTGGTTGGCGTGAACCATTTTTAAGTTCTGCGT
GTGACGGAGAGGAGGACGG
 
RHA0098 (SEQ ID NO: 8)
CCTGCACCCAGTGTCCCGTCGTTGGTGTTTTTTTGGCTTTTAGGGTAGGTGTGGCTT
GACGGAGAGGAGGACGG
 
RHA0144 (SEQ ID NO: 9)
CCTGCACCCAGTGTCCCTCGCTTCTTTTTTGGGTTGGGGTGGGCGGGTTCCTGTC
GGACGGAGAGGAGGACGG
 
RHA0133 (SEQ ID NO: 10)
CCTGCACCCAGTGTCCCCCTCTGTGTCGGGCGGGCCGTTCAGGGTTTTGGGTGGG
GGTGACGGAGAGGAGGACGG
 
RHA0020 (SEQ ID NO: 11)
GGTTAGCCCGTCCCGAGATAACCTGTGTTGGGGGTGGAGGGTTTTTGGGGTCTTA
ACACACGGCGGCTGTAG
 
RHA0002 (SEQ ID NO: 12)
GGTTAGCCCGTCCCGAGATAACTCGGGGTGTTTGATGGTTTGGTCTGGTTGGCTTA
 ACACACGGCGGCTGTAG RHA0002_s16 (SEQ ID NO: 13)
GGTTTGGTCTGGTTGG
RHA0002_s20 (SEQ ID NO: 14)
GTGGTTTGGTCTGGTTGGAC
RHA0002_s26 (SEQ ID NO: 15)
CGTTTGGTTTGGTCTGGTTGGCAACG
RHA0002_s28 (SEQ ID NO: 16)
CGTTATGGTTTGGTCTGGTTGGCTAACG
RHA0002_s33 (SEQ ID NO: 17)
GTGTTTGATGGTTTGGTCTGGTTGGCTTAACAC 
RHA0006 (SEQ ID NO: 18)
GGTTAGCCCGTCCCGAGATAACTCGGTGTGTTGGGTTTGGGTTGGGTTGGGTCTTA
ACACACGGCGGCTGTAG
 RHA0006_s19 (SEQ ID NO: 19)
GGGTTTGGGTTGGGTTGGG 
RHA1634 (SEQ ID NO: 20)
ATATATATGCCCACCCTCTCGCTGTACGGTTGATGCGCGTTTCTTTCTCTTTATATTG
CTGGGTGCCGCCTCCAAGGTCAAAAAAAA
 
RHA1634_s60 (SEQ ID NO: 21)
GGGCACCCTCTCGCTGTACGGTTGATGCACGTTTCTTTCTCTTTATATTGCTGGGTG
CCC
 
RHA1635 (SEQ ID NO: 22)
ATATATATGCCCACCCTCTCGCTGGCGGCTCTGTTCTGTTCTCGTCTCCTTGATTTCT
GTGGGCAGCCGCCTCCAAGGTCAAAAAAAA
 
RHA1635_s62 (SEQ ID NO: 23)
GGGGCCCACCCTCTCGCTGGCGGCTCTGTTCTGTTCTCGTCTCCTTGATTTCTGTGG
GCCCC
 
RHA0385 (SEQ ID NO: 24)
ATATATATCCTGCACCCAGTGTCCCGTGTGCAATTCAGTTGGGGTTATTTTGGGAGG
GCGGGGGTTGACGGAGAGGAGGACGGAAAAAAAA
 RHA0385_s28 (SEQ ID NO: 25)
TTGGGGTTATTTTGGGAGGGCGGGGGTT RHA0471 (SEQ ID NO: 26) 
CTGCACCCAGTGTCCCTTTCCTCGTTGGGGGTGGTGGTGGGTTTCGGTTCATGGGT
GGGACGGAGAGGAGGACGG
 RHA0471_s51 (SEQ ID NO: 27)
TCCTCGTTGGGGGTGGTGGTGGGTTTCGGTTCATGGGTGGGACGGAGAGGA

RHA0111 (SEQ ID NO: 28)
CCTGCACCCAGTGTCCCGTGCTCCGGGGGTTGGGCGTGGTGGGTCTGTCGGGTTT
CGGACGGAGAGGAGGACGG

RHA0127 (SEQ ID NO: 29)
CCTGCACCCAGTGTCCCTGGGTCGGCTAATTTGGCATTTGGGGTGGTTTGGGGGGG
GGACGGAGAGGAGGACGG

RHA0124 (SEQ ID NO: 30)
CCTGCACCCAGTGTCCCTGTGTGCTGGTGTGGGGTGGTTTGGGGGGGGGACGGAG
AGGAGGACGG

Regarding the polynucleotide (b), the term "one or more" is not limited as long as, for example, it is in the range where the polynucleotide (b) binds to the influenza virus or HA. The "one or more" bases in any of the base sequences of the polynucleotide (a) are, for example, 1 to 60 bases, preferably 1 to 30 bases, more preferably 1 to 15 bases, still more preferably 1 to 5 bases, and particularly preferably 1 or 2 bases.

Regarding the polynucleotide (c), the "identity" is not limited as long as, for example, it is in the range where the polynucleotide (c) binds to the influenza virus or HA. The identity is, for example, at least 80% or at least 85%, preferably at least 90%, more preferably at least 95%, at least 96%, or at least 97%, still more preferably at least 98%, and particularly preferably at least 99%. The identity can be calculated with analysis software such as BLAST or FASTA using default parameters, for example (the same applies hereinafter).

Regarding the polynucleotide (d), the "polynucleotide hybridizing to" is, for example, a polynucleotide perfectly or partially complementary to the polynucleotide (a). The hybridization can be detected by various kinds of hybridization assay, for example. The hybridization assay is not particularly limited, and for example, a method described in "Molecular Cloning: A Laboratory Manual 2nd Ed." edited by Sambrook et al. (Cold Spring Harbor Laboratory Press (1989)) or the like can be employed.

Regarding the polynucleotide (d), the "stringent conditions" may be, for example, any of low stringency conditions, medium stringency conditions, and high stringency conditions. The "low stringency conditions" are, for example, 5 × SSC, 5× Denhardt's solution, 0.5% SDS, 50% formamide, and 32°C. The "medium stringency conditions" are, for example, 5× SSC, 5 × Denhardt's solution, 0.5% SDS, 50% formamide, and 42°C. The "high stringency conditions" are, for example, 5× SSC, 5× Denhardt's solution, 0.5% SDS, and 50% formamide, and 50°C. Those skilled in the art can set the degree of stringency by, for example, setting the conditions such as the temperature, the salt concentration, the concentration and the length of a probe, the ionic strength, the time, etc. as appropriate. As the "stringent conditions", conditions described in "Molecular Cloning: A Laboratory Manual 2nd Ed." edited by Sambrook et al. (Cold Spring Harbor Laboratory Press (1989)) or the like described above can be employed, for example.

The polynucleotides (b) to (d) are not particularly limited. When the polynucleotide (a) has the base sequence of SEQ ID NO: 12 (RHA0002), the polynucleotides (b) to (d) may be, for example, polynucleotides having base sequences of SEQ ID NOs: 13 to 17. When the polynucleotide (a) has the base sequence of SEQ ID NO: 17 (RHA0002_s33), the polynucleotides (b) to (d) may be, for example, the polynucleotides having base sequences of SEQ ID NOs: 13 to 16.
RHA0002_s16 (SEQ ID NO: 13)
GGTTTGGTCTGGTTGG
RHA0002_s20 (SEQ ID NO: 14)
GTGGTTTGGTCTGGTTGGAC
RHA0002_s26 (SEQ ID NO: 15)
CGTTTGGTTTGGTCTGGTTGGCAACG
RHA0002_s28 (SEQ ID NO: 16)
CGTTATGGTTTGGTCTGGTTGGCTAACG
RHA0002_s33 (SEQ ID NO: 17)
GTGTTTGATGGTTTGGTCTGGTTGGCTTAACAC

The nucleic acid molecule according to the present invention may include, for example, one sequence selected from the polynucleotides (a) to (d) or a plurality of sequences selected from the polynucleotides (a) to (d). In the latter case, the plurality of polynucleotide sequences preferably are linked to each other to form a single-stranded polynucleotide. The plurality of polynucleotide sequences may be linked to each other directly, or may be linked to each other indirectly with a linker being present between each pair of adjacent polynucleotide sequences, for example. The polynucleotide sequences preferably are linked to each other directly or indirectly at their ends. The plurality of polynucleotide sequences may be the same or different from each other, for example. Preferably, the plurality of polynucleotide sequences are the same, for example. When the nucleic acid molecule of the present invention includes the plurality of polynucleotide sequences, the number of the sequences is not particularly limited, and is, for example, 2 or more, preferably 2 to 20, more preferably 2 to 10, and still more preferably 2 or 3.

The linker is not particularly limited. The length of the linker is not particularly limited, and is, for example, 1- to 200-mer, preferably 1- to 20-mer, more preferably 3- to 12-mer, and still more preferably 5- to 9-mer. The building blocks of the linker are, for example, nucleotide residues, examples of which include deoxyribonucleotide residues and ribonucleotide residues. The linker is not particularly limited, and examples thereof include polynucleotides such as DNA consisting of deoxyribonucleotide residues and DNA including a ribonucleotide residue(s). Specific examples of the linker include polydeoxythymine (poly(dT)), polydeoxyadenine (poly(dA)), and poly(dA-dT) having a repetitive sequence composed of A and T. Preferably, the linker is poly(dT) or poly(dA-dT).

Examples of a single-stranded polynucleotide including two sequences selected from the polynucleotides (a) to (d) include a polynucleotide including two base sequences of either SEQ ID NO: 19 (RHA0006_s19) or SEQ ID NO: 17 (RHA0002_s33) linked to each other via a linker. Examples of the sequence of the single-stranded polynucleotide are shown below as SEQ ID NO: 31 and SEQ ID NO: 45. In the following sequences, the underlined parts each correspond to the sequence of SEQ ID NO: 19 or SEQ ID NO: 33, and the sequence -(N)n-present between the sequences corresponds to a linker. N indicates a nucleotide residue. The nucleotide residue N is not particularly limited. The nucleotide residue N is, for example, a ribonucleotide residue or a deoxyribonucleotide residue, and preferably is a deoxyribonucleotide residue. The base in the nucleotide residue is not particularly limited, and is, for example, A, G, C, T, and/or U. In the following sequences, n is an integer that indicates the number of bases in the nucleotide residues. Preferably, n is a positive integer, and is, for example, 1 to 200 bases, preferably 5 to 20 bases.
RHA0006_s19_d (SEQ ID NO: 31)
GGGTTTGGGTTGGGTTGGG-(N)n-GGGTTTGGGTTGGGTTGGG

RHA0002_s33_d (SEQ ID NO: 45)
GTGTTTGATGGTTTGGTCTGGTTGGCTTAACAC-(N)n-GTGTTTGATGGTTTGGTCTG
GTTGGCTTAACAC

Specific examples of SEQ ID NO: 31 include the following sequences in which, for example, n = 5, n = 9, and n = 20.
RHA0006_s19_d9 (SEQ ID NO: 32)
GGGTTTGGGTTGGGTTGGGTTTTTTTTTGGGTTTGGGTTGGGTTGGG
RHA0006_s19_d5 (SEQ ID NO: 33)
GGGTTTGGGTTGGGTTGGGTTTTTGGGTTTGGGTTGGGTTGGG
RHA0006_s19_d9_AT (SEQ ID NO: 46)
GGGTTTGGGTTGGGTTGGGATATATATAGGGTTTGGGTTGGGTTGGG

RHA0006_s19_d_R20 (SEQ ID NO: 47)
GGGTTTGGGTTGGGTTGGGGATTGGAATTAAGGCGTGTGGGGTTTGGGTTGGGTT
GGG

Specific examples of SEQ ID NO: 45 include the following sequences in which, for example, n = 9 and n = 5.

RHA0002_s33_d9 (SEQ ID NO: 48)
GTGTTTGATGGTTTGGTCTGGTTGGCTTAACACTTTTTTTTTGTGTTTGATGGTTTG
GTCTGGTTGGCTTAACAC

RHA0002_s33_d5 (SEQ ID NO: 49)
GTGTTTGATGGTTTGGTCTGGTTGGCTTAACACTTTTTGTGTTTGATGGTTTGGTCT
GGTTGGCTTAACAC

Examples of a single-stranded polynucleotide including three sequences selected from the polynucleotides (a) to (d) include a polynucleotide including three base sequences of SEQ ID NO: 19 (RHA0006_s19) linked to each other via linkers. An example of the sequence of the single-stranded polynucleotide is shown below as SEQ ID NO: 50. In the following sequence, the underlined parts each correspond to the sequence of SEQ ID NO: 19, and the sequences -(N)n- between the sequences each correspond to a linker. N and n are as described above. In the following sequence, the sequences of the two linkers may be the same or different from each other, for example.

RHA0006_s19_t (SEQ ID NO: 50)
GGGTTTGGGTTGGGTTGGG-(N)n-GGGTTTGGGTTGGGTTGGG-(N)n-GGGTTTGGGT
TGGGTTGGG

Specific examples of SEQ ID NO: 50 include the following sequence in which, for example, n = 9.

RHA0006_s19_t9 (SEQ ID NO: 51)

GGGTTTGGGTTGGGTTGGGTTTTTTTTTGGGTTTGGGTTGGGTTGGGTTTTTTTTTG

GGTTTGGGTTGGGTTGGG

In the nucleic acid molecule of the present invention, the polynucleotide preferably is a single-stranded polynucleotide. It is preferable that the single-stranded polynucleotide can form a stem structure and a loop structure by self-annealing, for example. It is preferable that the polynucleotide can form a stem-loop structure, an internal loop structure, and/or a bulge structure, for example.

The nucleic acid molecule of the present invention may be a double strand, for example. When the nucleic acid molecule is a double strand, for example, one of single-stranded polynucleotides includes any of the polynucleotides (a) to (d), and the other single-stranded polynucleotide is not limited. The other single-stranded polynucleotide may be, for example, a polynucleotide including a base sequence complementary to any of the polynucleotides (a) to (d). When the nucleic acid molecule of the present invention is a double strand, it is preferable to dissociate the double strand to single-stranded polynucleotides by denaturation or the like before use, for example. Also, it is preferable that the dissociated single-stranded polynucleotide including any of the polynucleotides (a) to (d) is forming a stem structure and a loop structure as described above, for example.

In the present invention, the expression "can form a stem structure and a loop structure (and grammatical variations thereof)" encompasses that, for example, a stem structure and a loop structure are formed actually, and also, even if a stem structure and a loop structure are not formed, they can be formed depending on conditions. The expression "can form a stem structure and a loop structure (and grammatical variations thereof)" encompasses, for example, both the case where the formation thereof has been confirmed through an experiment and the case where the formation thereof is predicted through simulation using a computer or the like.

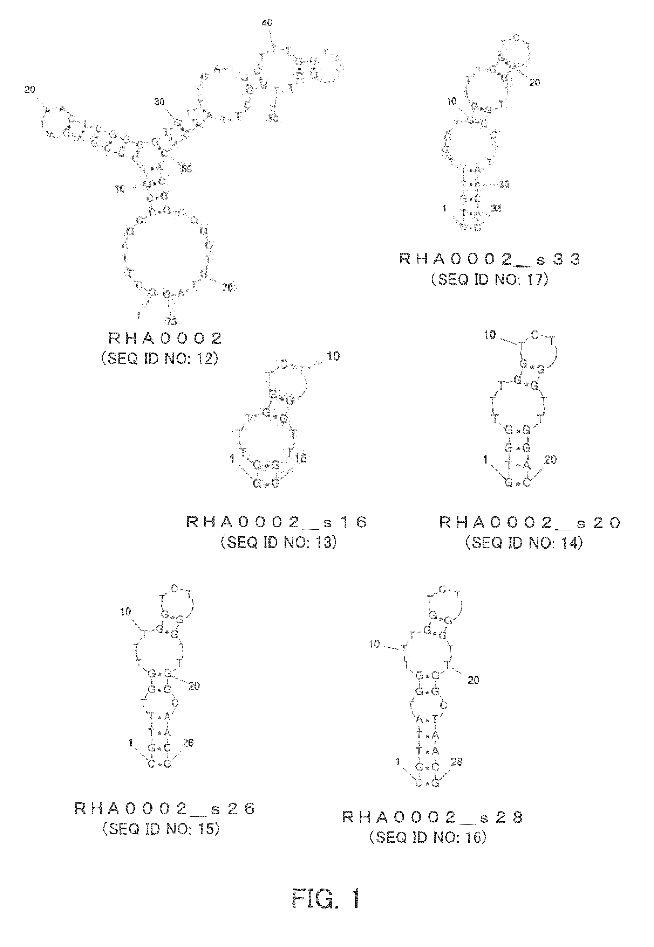

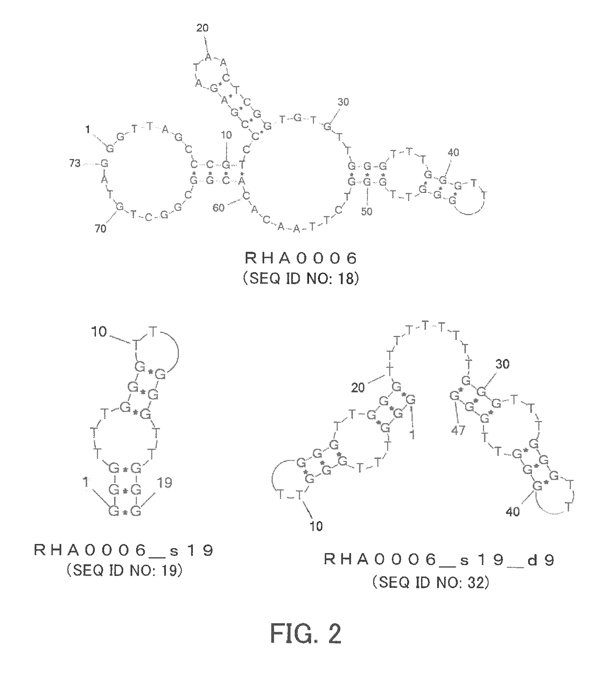

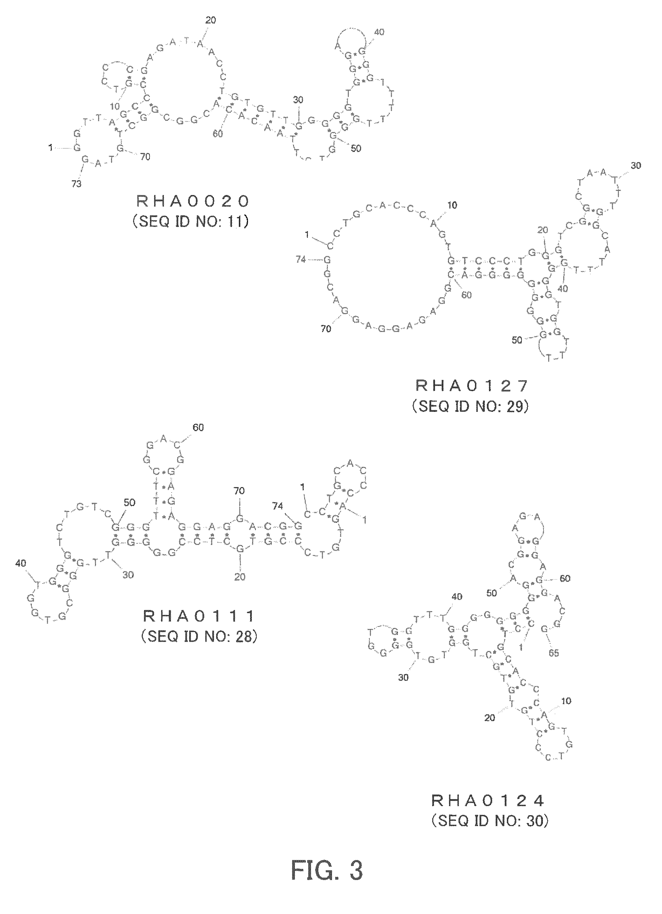

Predicted secondary structures of the polynucleotides are illustrated in FIGs. 1 to 3 and FIGs. 19 to 24. It is to be noted, however, that the present invention is not limited thereto. FIG. 1 shows predicted secondary structures of RHA0002 (SEQ ID NO: 12), RHA0002_s16 (SEQ ID NO: 13), RHA0002_s20 (SEQ ID NO: 14), RHA0002_s26 (SEQ ID NO: 15), RHA0002_s28 (SEQ ID NO: 16), and RHA0002_s33 (SEQ ID NO: 17). FIG 2 shows predicted secondary structures of RHA0006 (SEQ ID NO: 18), RHA0006_s19 (SEQ ID NO: 19), and RHA0006_s19_d9 (SEQ ID NO: 32) in which the linker is poly(dT) consisting of 9 bases. FIG. 3 shows predicted secondary structures of RHA0020 (SEQ ID NO: 11), RHA0111 (SEQ ID NO: 28), RHA0127 (SEQ ID NO: 29), and RHA0124 (SEQ ID NO: 30).

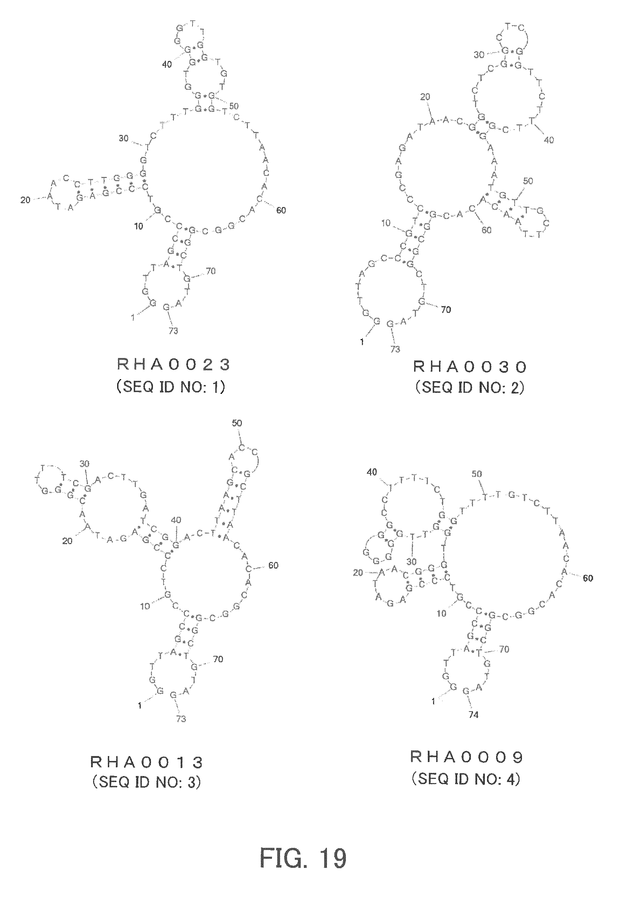

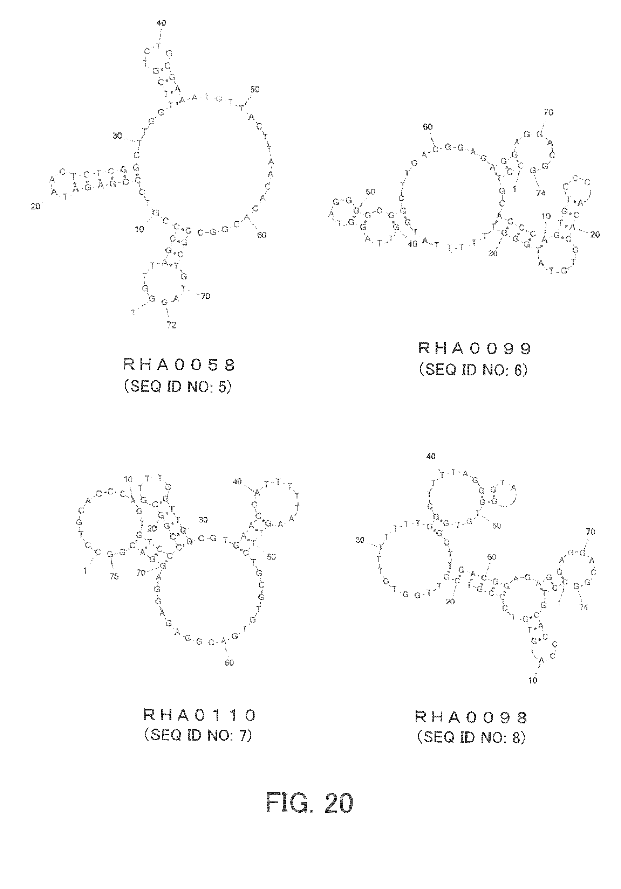

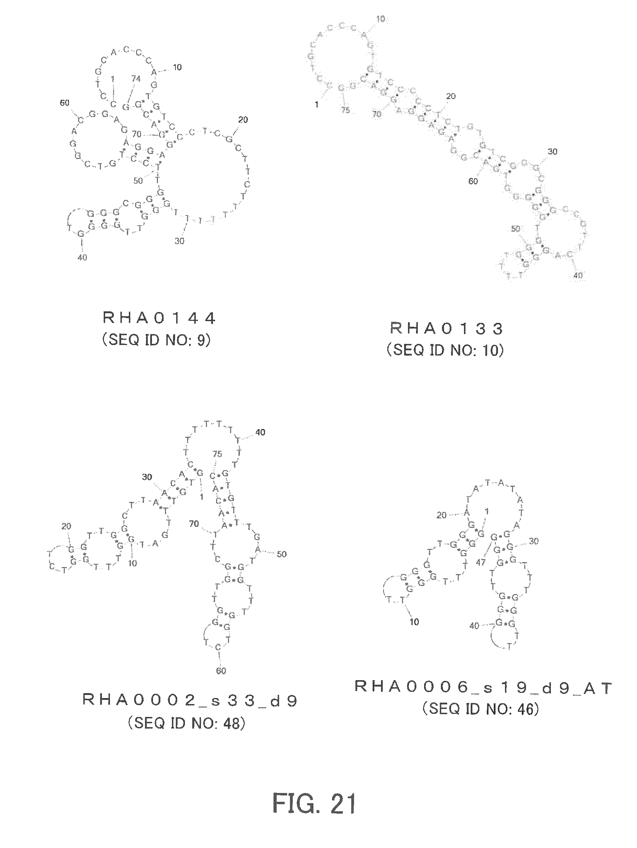

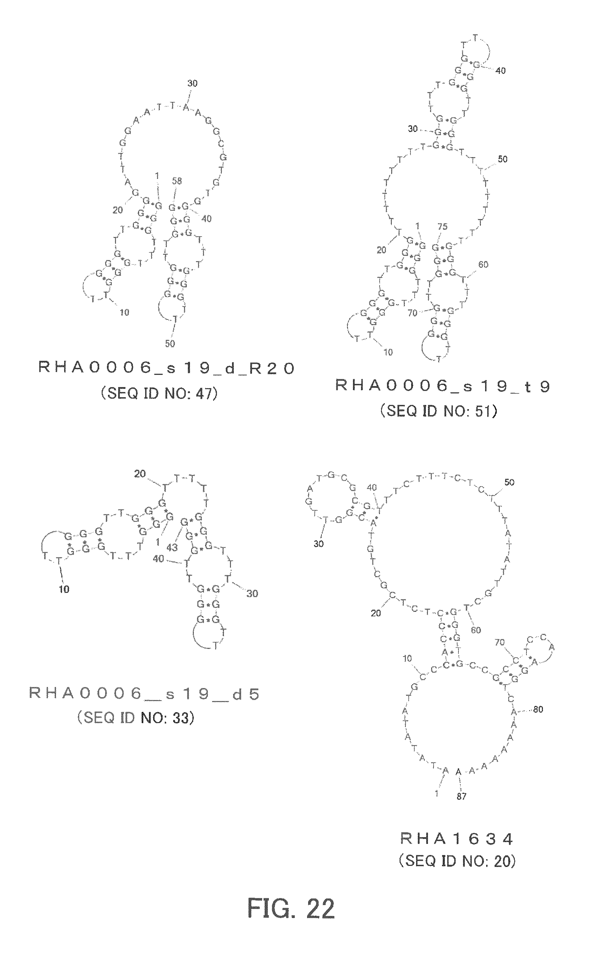

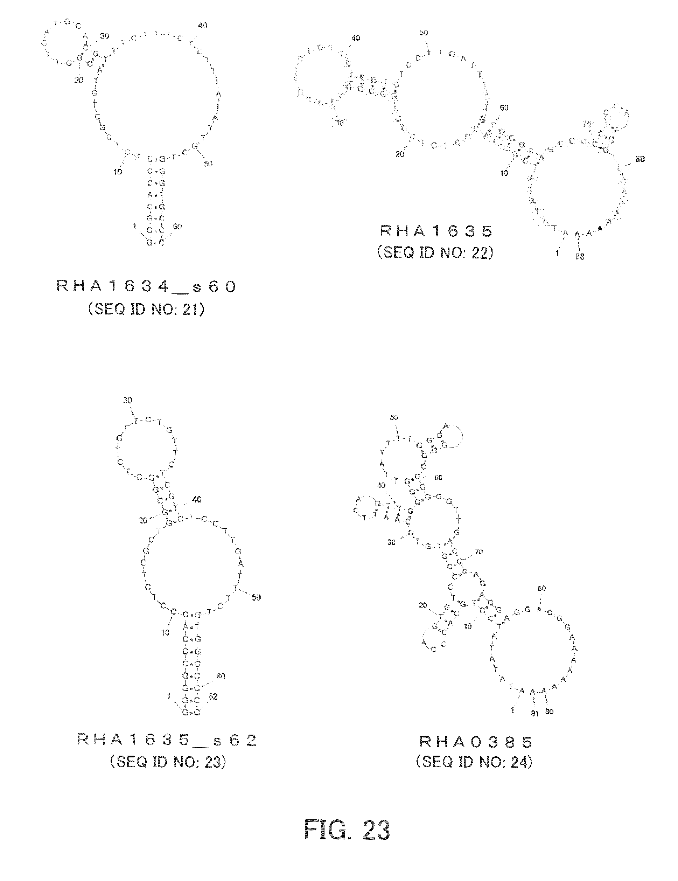

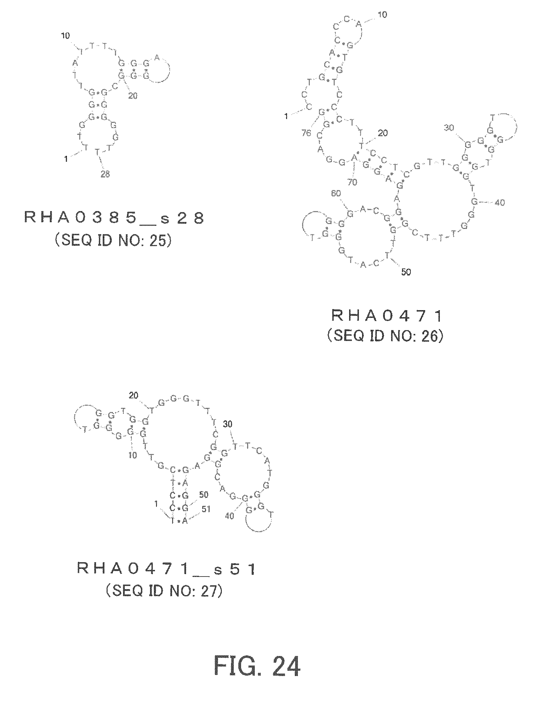

FIG. 19 shows predicted secondary structures of RHA0023 (SEQ ID NO: 1), RHA0030 (SEQ ID NO: 2), RHA0013 (SEQ ID NO: 3), and RHA0009 (SEQ ID NO: 4). FIG. 20 shows predicted secondary structures of RHA0058 (SEQ ID NO: 5), RHA0099 (SEQ ID NO: 6), RHA0110 (SEQ ID NO: 7), and RHA0098 (SEQ ID NO: 8). FIG 21 shows predicted secondary structures of RHA0144 (SEQ ID NO: 9), RHA0133 (SEQ ID NO: 10), RHA0002_s33_d9 (SEQ ID NO: 48), and RHA0006_s19_d9_AT(SEQ ID NO: 46). FIG. 22 shows predicted secondary structures of RHA0006_s19_d_R20 (SEQ ID NO: 47), RHA0006_s19_t9 (SEQ ID NO: 51), RHA0006_s19_d5 (SEQ ID NO: 33), and RHA1634 (SEQ ID NO:20). FIG 23 shows predicted secondary structures of RHA1634_s60 (SEQ ID NO: 21), RHA1635 (SEQ ID NO: 22), RHA1635_s62 (SEQ ID NO: 23), and RHA0385 (SEQ ID NO: 24). FIG. 24 shows predicted secondary structures of RHA0385_s28 (SEQ ID NO: 25), RHA0471 (SEQ ID NO: 26), and RHA0471_s51 (SEQ ID NO: 27).

The building blocks of the nucleic acid molecule of the present invention are, for example, nucleotide residues. Examples of the nucleotide residues include deoxyribonucleotide residues and ribonucleotide residues. Examples of the nucleic acid molecule of the present invention include DNA consisting of deoxyribonucleotide residues only and DNA including one or more ribonucleotide residues. In the latter case, "one or more" is not particularly limited. For example, the number of the ribonucleotide residues in the polynucleotide is, for example, 1 to 91, preferably 1 to 30, more preferably 1 to 15, still more preferably 1 to 7, particularly preferably 1 to 3, and most preferably 1 or 2.

The nucleic acid molecule of the present invention may include one or more modified nucleotide residues, for example. The term "one or more" is not particularly limited. For example, the number of the modified nucleotide residues in the polynucleotide is, for example, 1 to 91, preferably 1 to 30, more preferably 1 to 15, still more preferably 1 to 7, particularly preferably 1 to 3, and most preferably 1 or 2.

Examples of the modified nucleotide residue include modified deoxyribonucleotide residues and modified ribonucleotide residues. The modified nucleotide residue may be the above-described nucleotide residue with a modified sugar residue, for example. Examples of the sugar residue include ribose residues and deoxyribose residues. The modified site in the nucleotide residue is not particularly limited, and may be, for example, the 2'-position and/or the 4'-position in the sugar residue. Examples of the modification include methylation, fluorination, amination, and thiation. The modified nucleotide residue may be, for example, the one obtained by modification of a nucleotide residue having a pyrimidine base (pyrimidine nucleus) as the base or the one obtained by modification of a nucleotide residue having a purine base (purine nucleus) as the base. Among them, the former is preferable. Hereinafter, the nucleotide residue having a pyrimidine base is referred to as a "pyrimidine nucleotide residue"; a pyrimidine nucleotide residue that has been modified is referred to as a "modified pyrimidine nucleotide residue"; a nucleotide residue having a purine base is referred to as a "purine nucleotide residue"; and a purine nucleotide residue that has been modified is referred to as a "modified purine nucleotide residue". Examples of the pyrimidine nucleotide residue include: uracil nucleotide residues having uracil; cytosine nucleotide residues having cytosine; and thymine nucleotide residues having thymine. In the case where the base in the modified nucleotide residue is a pyrimidine base, for example, it is preferable that the 2'-position and/or carbon in the 4'-position in the sugar residue is modified. Specific examples of the modified nucleotide residue include modified nucleotide residues with the 2'-position in the ribose residue being modified, such as a 2'-methylated-uracil nucleotide residue, a 2'-methylated-cytosine nucleotide residue, a 2'-fluorinated-uracil nucleotide residue, a 2'-fluorinated-cytosine nucleotide residue, a 2'-aminated-uracil nucleotide residue, a 2'-aminated-cytosine nucleotide residue, a 2'-thiated-uracil nucleotide residue, and a 2'-thiated-cytosine nucleotide residue.

The base in the nucleotide residue may be, for example, a natural base (non-artificial base) such as adenine (a), cytosine (c), guanine (g), thymine (t), or uracil (u), or an unnatural base (artificial base). Examples of the artificial base include a modified base and an altered base, which both preferably has a similar function to the natural base (a, c, g, t, or u). Examples of the artificial base having the similar function include: an artificial base that can bind to cytosine (c), as a substitute for guanine (g); an artificial base that can bind to guanine (g), as a substitute for cytosine (c); an artificial base that can bind to thymine (t) or uracil (u), as a substitute for adenine (a); an artificial base that can bind to adenine (a), as a substitute for thymine (t); and an artificial base that can bind to adenine (a), as a substitute for uracil (u). Examples of the modified base include methylated bases, fluorinated bases, aminated bases, and thiated bases. Specific examples of the modified base include 2'-methyluracil, 2'-methylcytosine, 2'-fluorouracil, 2'-fluorocytosine, 2'-aminouracil, 2'-aminocytosine, 2'-thiouracil, and 2'-thiocytosine. In the present invention, for example, bases represented by a, g, c, t, and u encompass not only the natural bases but also the artificial bases having similar functions to the natural bases.

The nucleic acid molecule of the present invention may include one or more artificial nucleic acid monomer residues, for example. The term "one or more" is not particularly limited. For example, the number of the artificial nucleic acid monomer residues in the polynucleotide is, for example, 1 to 91, preferably 1 to 30, more preferably 1 to 15, still more preferably 1 to 7, particularly preferably 1 to 3, and most preferably 1 or 2. Examples of the artificial nucleic acid monomer residue include PNA (peptide nucleic acid), LNA (Locked Nucleic Acid), and ENA (2'-O, 4'-C-Ethylenebridged Nucleic Acids). The nucleic acid in the monomer residue is as described above, for example.

It is preferable that the nucleic acid molecule of the present invention is resistant to nuclease, for example. In order to allow the nucleic acid molecule to have nuclease resistance, it is preferable that the nucleic acid molecule of the present invention includes the modified nucleotide residue(s) and/or the artificial nucleic acid monomer residue(s), for example. Also, in order to allow the nucleic acid molecule to have nuclease resistance, the nucleic acid molecule of the present invention may have PEG (polyethylene glycol) of several tens of kDa, deoxythymidine, or the like bound to, e.g., the 5' end or the 3' end thereof.

The nucleic acid molecule of the present invention may further include an additional sequence, for example. Preferably, the additional sequence is bound to at least one of the 5' end and the 3' end, more preferably to the 3' end of the nucleic acid molecule, for example. The additional sequence is not particularly limited. The length of the additional sequence is not particularly limited, and is, for example, 1- to 200-mer, preferably 1- to 50-mer, more preferably 1- to 25-mer, and still more preferably 18- to 24-mer. The building blocks of the additional sequence are, for example, nucleotide residues, examples of which include deoxyribonucleotide residues and ribonucleotide residues. The additional sequence is not particularly limited, and examples thereof include polynucleotides such as DNA consisting of deoxyribonucleotide residues and DNA including a ribonucleotide residue(s). Specific examples of the additional sequence include poly(dT) and poly(dA), and the additional sequence preferably is poly(dT) or poly(dA).

The nucleic acid molecule of the present invention can be used in the state where it is immobilized on a carrier, for example. It is preferable to immobilize either the 5' end or the 3' end, more preferably the 3' end of the nucleic acid molecule of the present invention, for example. When the nucleic acid molecule of the present invention is immobilized, the nucleic acid molecule may be immobilized either directly or indirectly to the carrier, for example. In the latter case, it is preferable to immobilize the nucleic acid molecule via the additional sequence, for example. The method for immobilizing the nucleic acid molecule is not particularly limited. For example, streptavidin or avidin is bound to either one of the carrier and the nucleic acid molecule/the additional sequence, and biotin is bound to the other. By utilizing the binding of between the former and the latter, immobilization can be achieved. The carrier is not particularly limited, and may be, for example, a base such as a plate, a sheet, a film, a filter, a tube, or beads.

The method for producing the nucleic acid molecule of the present invention is not particularly limited. For example, the nucleic acid molecule of the present invention can be synthesized by known methods such as nucleic acid synthesis methods etc. utilizing chemical synthesis and methods utilizing genetic engineering.

The dissociation constant of the nucleic acid molecule of the present invention against an influenza virus or the HA is, for example, 30 nmol/L or less, preferably 10 nmol/L or less, more preferably 1 nmol/L or less, still more preferably 0.1 nmol/L or less, and particularly preferably 0.01 nmol/L or less.

The nucleic acid molecule of the present invention exhibits binding properties to influenza viruses or influenza virus-derived HA, as described above. Thus, use of the nucleic acid molecule of the present invention is not particularly limited as long as it is the use utilizing the binding properties to the influenza viruses and the HA. The nucleic acid molecule of the present invention can be used in various methods, for example, as a substitute for an antibody against the influenza viruses and the HA.

According to the nucleic acid molecule of the present invention, for example, by detecting the binding between the nucleic acid molecule and an influenza virus, it is possible to conduct analysis such as qualification or quantification with respect to the influenza virus. That is, a method for analyzing an influenza virus according to the present invention is characterized in that it includes the steps of: bringing the nucleic acid molecule of the present invention into contact with a sample; and detecting the binding between the nucleic acid molecule and an influenza virus. More specifically, for example, the presence or absence of the influenza virus in the sample can be analyzed by detecting the presence or absence of the binding between the nucleic acid molecule and the influenza virus in the detection step, or alternatively, the amount of the influenza virus in the sample can be analyzed by measuring the amount of the binding between the nucleic acid molecule and the influenza virus in the sample. According to this method, for example, because the nucleic acid molecule of the present invention can bind to influenza viruses, an influenza virus in a sample can be analyzed without subjecting the sample to a pretreatment for, e.g., destroying the virus.

Also, according to the nucleic acid molecule of the present invention, for example, by detecting the binding between the nucleic acid molecule and HA derived from an influenza virus, it is possible to conduct analysis such as qualification or quantification with respect to the HA or indirectly with respect to the influenza virus. That is, a method for analyzing influenza virus-derived HA according to the present invention is characterized in that it includes the steps of: bringing the nucleic acid molecule of the present invention into contact with a sample; and detecting the binding between the nucleic acid molecule and the HA. More specifically, in this analysis method, the presence or absence of the HA in the sample can be analyzed by detecting the presence or absence of the binding between the nucleic acid molecule and the HA in the detection step, or alternatively, the amount of the HA in the sample can be analyzed by measuring the amount of the binding between the nucleic acid molecule and the HA in the sample. Furthermore, it is also possible to carry out influenza virus detection by utilizing this HA detection method. An influenza virus detection method according to the present invention includes, for example, the above-described HA analysis method, and it analyzes an influenza virus in the sample by detecting the binding between the nucleic acid molecule and the HA. More specifically, the presence or absence of the influenza virus in the sample can be analyzed by detecting the presence or absence of the binding between the nucleic acid molecule and the HA in the detection step, or alternatively, the amount of the influenza virus in the sample can be analyzed by measuring the amount of the binding between the nucleic acid molecule and the HA in the sample. The amount of HA and the amount of an influenza virus generally are in a relative relationship. Thus, for example, by determining the relative relationship between the amount of the influenza virus and the amount of the HA in advance, the amount of the influenza virus can be analyzed from the amount of the HA on the basis of the thus-determined relative relationship. The relative relationship can be indicated with a calibration curve or the like, for example. In the case of this method, for example, the nucleic acid molecule may be brought into contact with the sample after the sample has been subjected to a pretreatment of destroying the virus, or the nucleic acid molecule may be brought into contact with the sample without conducting the pretreatment.

The nucleic acid molecule of the present invention can bind to influenza viruses, as described above. Thus, the nucleic acid molecule of the present invention can be used widely in, for example, not only the above-described influenza virus detection and HA detection but also various uses that utilize their binding. Specific examples of such uses include products containing the nucleic acid molecule, such as a filter, a mask, a wiping agent, a gargling agent, and a troche. Examples of the filter include an air filter and a liquid filter. The nucleic acid molecule in the filter binds to influenza viruses. Thus, the filter can remove the influenza viruses from the air and liquid and can prevent the spread of the influenza viruses, for example. The mask can prevent infection with influenza viruses, because the nucleic acid molecule binds to the influenza viruses to inhibit the invasion of the influenza viruses into the body. The wiping agent can remove influenza viruses from an object to be wiped using the wiping agent, because the nucleic acid molecule binds to the influenza viruses, for example. The gargling agent can remove influenza viruses attached to the oral cavity and the pharynx, because the nucleic acid molecule binds to the influenza viruses, for example.

### <Binding Agent>

As described above, the binding agent according to the present invention is a binding agent that binds to an influenza virus, characterized in that it contains the nucleic acid molecule of the present invention. It is only necessary that the binding agent according to the present invention contains the nucleic acid molecule according to the present invention, and other configurations are by no means limited. By using the binding agent of the present invention, detection and the like of influenza viruses are possible, for example, as described above. Also, as described above, the binding agent of the present invention also can be referred to as, for example, a binding agent that binds to influenza virus-derived HA, and it is possible to detect an influenza virus by detecting the HA, for example.

### Examples

Next, examples of the present invention will be described. It is to be noted, however, that the present invention is by no means limited by the following examples. Commercially available reagents in the examples were used in accordance with their protocols, unless otherwise stated.

### [Example A1]

Aptamers capable of binding to influenza viruses were prepared, and the binding ability of each aptamer to influenza virus-derived HA was examined.

### (1) Aptamers

As aptamers of the present example, the following polynucleotides were synthesized.

RHA0002 (SEQ ID NO: 12)
GGTTAGCCCGTCCCGAGATAACTCGGGGTGTTTGATGGTTTGGTCTGGTTGGCTTA
ACACACGGCGGCTGTAG

RHA0006 (SEQ ID NO: 18)
GGTTAGCCCGTCCCGAGATAACTCGGTGTGTTGGGTTTGGGTTGGGTTGGGTCTTA
ACACACGGCGGCTGTAG

RHA0111 (SEQ ID NO: 28)
CCTGCACCCAGTGTCCCGTGCTCCGGGGGTTGGGCGTGGTGGGTCTGTCGGGTTT
CGGACGGAGAGGAGGACGG

RHA0127 (SEQ ID NO: 29)
CCTGCACCCAGTGTCCCTGGGTCGGCTAATTTGGCATTTGGGGTGGTTTGGGGGGG
GGACGGAGAGGAGGACGG

A DNA library containing a plurality of DNAs each consisting of an oligonucleotide represented by SEQ ID NO: 34, which includes a 30-mer random sequence (N)₃₀, was prepared as Comparative Example N30. Also, a DNA library containing a plurality of DNAs each consisting of an oligonucleotide represented by SEQ ID NO: 35, which includes a 40-mer random sequence (N)₄₀, was provided as Comparative Example N40. In the following sequences, "N" denotes deoxyribonucleotide residues, and nucleic acids contained therein are adenine, guanine, cytosine, and/or thymine.
N30 (SEQ ID NO: 34)
GGTTAGCCCGTCCCGAGATAAC-(N)₃₀-CTTAACACACGGCGGCTGTAG
N40 (SEQ ID NO: 35)
ACCCAGTGTCCC-(N)₄₀-GACGGAGAGGAGGACGG

As an aptamer A10 according to a comparative example, a polynucleotides having the following sequence described in Non-Patent Document 2 (BBRC) was synthesized.

Aptamer A10 (SEQ ID NO: 52)
GAATTCAGTCGGACAGCGGGGTTCCCATGCGGATGTTATAAAGCAGTCGCTTATAA
GGGATGGACGAATATCGTCTCCC

To the 3' end of each of the above-described aptamers, 24-mer polydeoxyadenine (poly(dA)) was added. The thus-obtained poly(dA)-added aptamers were used in SPR to be described below.

### (2) Target proteins

As target proteins, the following proteins were used. A/Anhui/1/2005-derived HA1 and A/goose_Guiyang/337/2006-derived HA1 are composed of 437 amino acid residues, and 24 amino acid residues out of the 437 amino acid residues are different from each other (5.49%). In SEQ ID NOs: 36 and 37, different amino acids are underlined. In SEQ ID NO: 38 of A/Anhui/1/2005-derived HA, amino acids different from those in A/goose_Guiyang/337/2006-derived HA are underlined.

### BSA

bovine serum albumin
Nippon Gene Co., Ltd.

### His-MIF

MIF (macrophage migration inhibitory factor) with a histidine tag added thereto
ATGen Co., Ltd. Gyeonggi-do

### HA1_{Anhui}

A/Anhui/1/2005-derived HA1
Sino Biological Inc. (#11048-V08H2)

SEQ ID NO: 36
MEKIVLLLAIVSLVKSDQICIGYHANNSTEQVDTIMEKNVTVTHAQDILE
KTHNGKLCDLDGVKPLILRDCSVAGWLLGNPMCDEFINVPEWSYIVEKAN

PANDLCYPGNFNDYEELKHLLSRINHFEKIQIIPKSSWSDHEASSGVSSA
CPYQGTPSFFRNVVWLIKKNNTYPTIKRSYNNTNQEDLLILWGIHHSNDA
AEQTKLYQNPTTYISVGTSTLNQRLVPKIATRSKVNGQSGRMDFFWTILK
PNDAINFESNGNFIAPEYAYKIVKKGDSAIVKSEVEYGNCNTKCQTPIGA
INSSMPFHNIHPLTIGECPKYVKSNKLVLATGLRNSP

### HA1_{Guiyang}

A/goose_Guiyang/337/2006-derived HA1
Sino Biological Inc. (#11690-V08H1)

SEQ ID NO: 37
MEKIVLLLAIISLVKSDQICIGYHANNSTVQVDTIMEKNVTVTHAQDILE
KTHNGKLCSLDGVKPLILRDCSVAGWLLGNPMCDEFINVPEWSYIVEKAS
PANDLCYPGDFNDYEELKHLLSRINHFEKIQIIPKSSWPNHEASLGVSSA
CPYQGESSFFRNVVWLIKKNSSYPTIKRSYNNTNQEDLLVLWGIHHPNDA
AEQIKLYQNPNTYISVGTSTLNQRLVPTIATRSKVNGQSGRMEFFWTILK
PNDAINFESNGNFIAPEYAYKIVKKGDSAIMKSELEYGNCNTKCQTPMIGA
INSSMPFHNIHPLTIGECPKYVKSNRLVLATGLRNTL

### HA_{Anhui}

A/Anhui/1/2005-derived HA
Sino Biological Inc. (#11048-V08H1)

SEQ ID NO: 38
MEKIVLLLAIVSLVKSDQICIGYHANNSTEQVDTIMEKNVTVTHAQDILE
KTHNGKLCDLDGVKPLILRDCSVAGWLLGNPMCDEFINVPEWSYIVEKAN
PANDLCYPGNFNDYEELKHLLSRINHFEKIQIIPKSSWSDHEASSGVSSA
CPYQGTPSFFRNVVWLIKKNNTYPTIKRSYNNTNQEDLLILWGIHHSNDA
AEQTKLYQNPTTYISVGTSTLNQRLVPKIATRSKVNGQSGRMDFFWTILK
PNDAINFESNGNFIAPEYAYKIVKKGDSAIVKSEVEYGNCNTKCQTPIGA
INSSMPFHNIHPLTIGECPKYVKSNKLVLATGLRNSP
RGLFGAIAGFIEGGWQGMVDGWYGYHHSNEQGSGYAADKESTQKAIDGVT
NKVNSIIDKMNTQFEAVGREFNNLERRIENLNKKMEDGFLDVWTYNAELL
VLMENERTLDFHDSNVKNLYDKVRLQLRDNAKELGNGCFEFYHKCDNECM
ESVRNGTYDYPQYSEEARLKREEISGVKLESIGTYQ

### (3) Analysis of binding ability by SPR

The analysis of the binding ability was carried out using a ProteON XPR36 (BioRad) in accordance with its instructions for use.

First, as a sensor chip designed specifically for the ProteON, a streptavidin-immobilized chip (trade name: ProteOn NLC Sensor Chip, BioRad) was set in the ProteON XPR36. A ligand at 5000 nmol/L was injected to a flow cell of the sensor chip using ultrapure water (DDW), and the binding was allowed to proceed until the signal intensity (RU: Resonance Unit) reached about 1000 RU. As the ligand, biotinylated poly(dT) obtained by biotinylating the 5' end of 24-mer deoxythymidine was used. Then, the poly(dA)-added aptamer at 400 nmol/L was injected to the flow cell of the chip using an SPR buffer at a flow rate of 25 µL/min for 80 seconds, and the binding was allowed to proceed until the signal intensity reached about 700 RU. Subsequently, the target protein at 500 nmol/L was injected using the SPR buffer at a flow rate of 50 µL/min for 120 seconds, followed by washing performed by flowing the SPR buffer under the same conditions. Signal intensity measurement was performed concurrently with the injection of the target protein and the washing with the SPR buffer.

A selection buffer had the following composition: 50 mmol/L Tris, 0.1 mol/L NaCl, 0.01 % Tween®, 5 mmol/L K⁺, and 1 mmol/L Mg²⁺. The pH of the selection buffer was 7.4. The SPR buffer had the following composition: 50 mmol/L Tris, 0.1 mol/L NaCl, 0.01 % Tween®, and 10 mmol/L K⁺. The pH of the SPR buffer was 7.4.

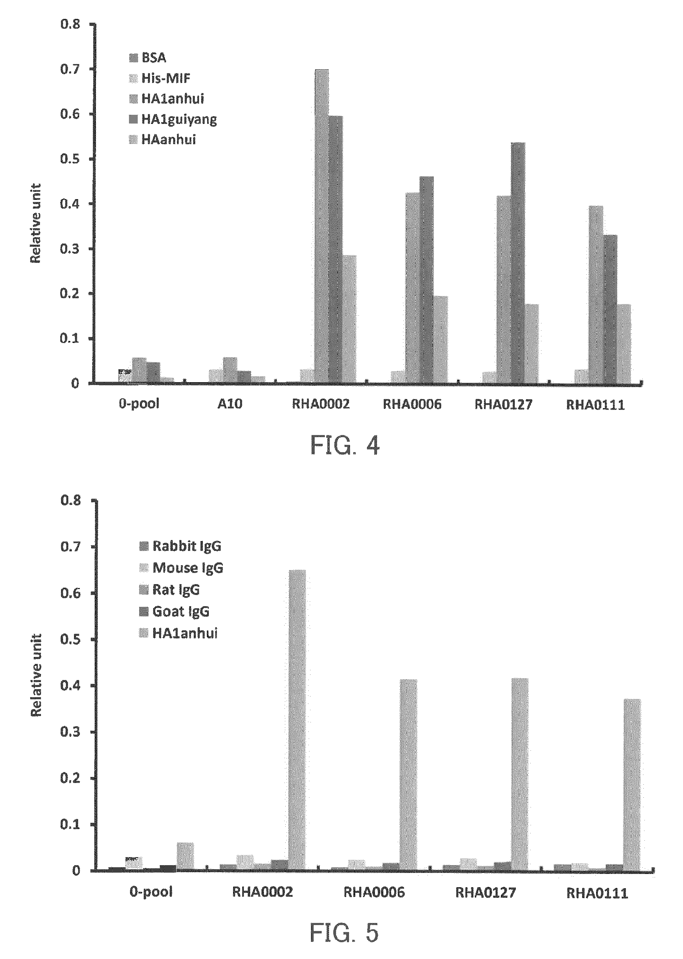

The results thereof are shown in FIG. 4. FIG. 4 is a graph showing the binding ability of each aptamer to each target protein. In the graph of FIG 4, the vertical axis indicates the relative value of the signal intensity (RU) measured using the Prote ON® XRP36. The relative value was determined by calculating a value obtained by dividing the signal intensity at the start of the washing (at the end of the target protein injection) with the signal intensity before the start of the target protein injection (at the end of the poly(dA)-added aptamer injection). In FIG. 4, "0-pool" indicates the result obtained regarding the N30.

As can be seen from FIG. 4, the aptamers of the comparative examples both did not exhibit binding ability to HA1_{Anhui}, HA1_{Guiyang}, and HA_{Anhui}. In contrast, the aptamers of the present example all exhibited binding ability to HA1_{Anhui}, HA1_{Guiyang}, and HA_{Anhui}, whereas they did not bind to either BSA or His-MIF.

### (4) Cross-reaction

Next, cross-reactions of each of the aptamers with antibodies were examined. SPR was carried out in the same manner as in the above item (3), except that HA1_{Anhui} and the following antibodies were used as the target proteins.

### Rabbit IgG

### Rabbit immunoglobulin

Beckman Coulter, Inc. (#731642)

### Mouse IgG

Mouse immunoglobulin
Beckman Coulter, Inc. (#731620)

### Rat IgG

Rat immunoglobulin
Beckman Coulter, Inc. (#731628)

### Goat IgG

Goat immunoglobulin
Beckman Coulter, Inc. (#731635)

### Chicken IgG

Chicken immunoglobulin
Immunology Consultants Laboratory (ICL), Inc. (#CGHL-10A)

The results thereof are shown in FIG. 5. FIG. 5 is a graph showing the binding ability of each aptamer to each antibody. In the graph of FIG. 5, the vertical axis indicates the relative value of the signal intensity (RU), as in FIG. 4. The relative value was determined in the same manner as in the item (3) above. In FIG. 5, "0-pool" indicates the result obtained regarding the N30.

As can be seen from FIG. 5, the aptamers of the present example exhibited potent binding ability to HA1_{Anhui}, whereas they did not exhibit binding ability to any of the antibodies. These results demonstrate that, for example, at the time of detecting an influenza virus or HA using the aptamer of the present invention, an antibody also can be used in combination.

### [Example A2]

The present example examined the binding ability of an aptamer obtained by truncating RHA0002 (SEQ ID NO: 12) to HA.

### (1) Aptamers

RHA0002 (SEQ ID NO: 12) and a truncated aptamer thereof shown below were synthesized. 
RHA0002 (SEQ ID NO: 12)
GGTTAGCCCGTCCCGAGATAACTCGGGGTGTTTGATGGTTTGGTCTGGTTGGCTTA
ACACACGGCGGCTGTAG
 RHA0002_s33 (SEQ ID NO: 17)
GTGTTTGATGGTTTGGTCTGGTTGGCTTAACAC

### (2) Analysis of binding ability by SPR

Except that the above aptamers were used, the binding ability to target proteins was analyzed in the same manner as in Example A1. The results thereof are shown in Table 1 below. In Table 1, the relative value was determined in the same manner as in the item (3) in Example A1.

**[Table 1]**

| Aptamer | Relative Value | | |
|---|---|---|---|
| | HA1_{Anhui} | HA1_{Guiyang} | HA_{Anhui} |
| RHA0002 | 0.5039 | 0.4935 | 0.2936 |
| RHA0002_s33 | 0.9958 | 0.9477 | 0.9847 |
| N30 | 0.0286 | 0.0091 | 0.0045 |

As can be seen from Table 1, the truncated aptamer exhibited binding ability to each target protein. In particular, RHA0002_s33 (SEQ ID NO: 17) exhibited improved binding ability by truncation.

### [Example A3]

The present example examined the binding ability to HA of: RHA0006 (SEQ ID NO: 18); a truncated aptamer obtained by truncating RHA0006, and an aptamer including two truncated sequences of the truncated aptamer.

### (1) Aptamers

RHA0006 (SEQ ID NO: 18)
GGTTAGCCCGTCCCGAGATAACTCGGTGTGTTGGGTTTGGGTTGGGTTGGGTCTTA
ACACACGGCGGCTGTAG

RHA0006_s19 (SEQ ID NO: 19)
GGGTTTGGGTTGGGTTGGG
RHA0006_s19_d9 (SEQ ID NO: 32)
GGGTTTGGGTTGGGTTGGGTTTTTTTTTGGGTTTGGGTTGGGTTGGG

### (2) Analysis of binding ability by SPR

Except that the above aptamers were used, the binding ability to target proteins was analyzed in the same manner as in Example A1. The results thereof are shown in Table 2 below. In Table 2, the relative value was determined in the same manner as in the item (3) in Example A1.

**[Table 2]**

| Aptamer | Relative Value | | |
|---|---|---|---|
| | HA1_{Anhui} | HA1_{Guiyang} | HA_{Anhui} |
| RHA0006 | 0.2970 | 0.2432 | 0.1122 |
| RHA0006_s19 | 0.4240 | 0.2297 | 0.1662 |
| RHA0006_s19_d | 0.9374 | 0.9096 | 0.7082 |
| N30 | 0.0286 | 0.0091 | 0.0045 |

As can be seen from Table 2, the truncated aptamer and the aptamers having the two truncated sequences each exhibited binding ability to the respective target proteins. In particular, RHA0006_s19_d9 (SEQ ID NO: 32), which is the aptamer having the two truncated sequences, exhibited further improved binding ability.

[Example A4]

The present example examined the binding ability of each aptamer to HA.

### (1) Aptamers

As aptamers of the present example, the following polynucleotides were synthesized. 
RHA0002 (SEQ ID NO: 12)
GGTTAGCCCGTCCCGAGATAACTCGGGGTGTTTGATGGTTTGGTCTGGTTGGCTTA
ACACACGGCGGCTGTAG
 RHA0002_s33 (SEQ ID NO: 17)
GTGTTTGATGGTTTGGTCTGGTTGGCTTAACAC 
RHA0006 (SEQ ID NO: 18)
GGTTAGCCCGTCCCGAGATAACTCGGTGTGTTGGGTTTGGGTTGGGTTGGGTCTTA
ACACACGGCGGCTGTAG
 RHA0006_s19 (SEQ ID NO: 19)
GGGTTTGGGTTGGGTTGGG
RHA0006_s19_d9 (SEQ ID NO: 32)
GGGTTTGGGTTGGGTTGGGTTTTTTTTTGGGTTTGGGTTGGGTTGGG

As aptamers of comparative examples, the aptamer A10 used as the aptamer of the comparative example in Example A1 and an aptamer A05 having the following sequence described in Non-Patent Document 2 (BBRC) were used.

Comparative Example A05 (SEQ ID NO: 53)
GAATTCAGTCGGACAGCGGGGGGAATTCTAGCAGTACACCTCGAGAATTATAGCC
AGGATGGACGAATATCGTCTCCC

### (2) Analysis of binding ability by SPR

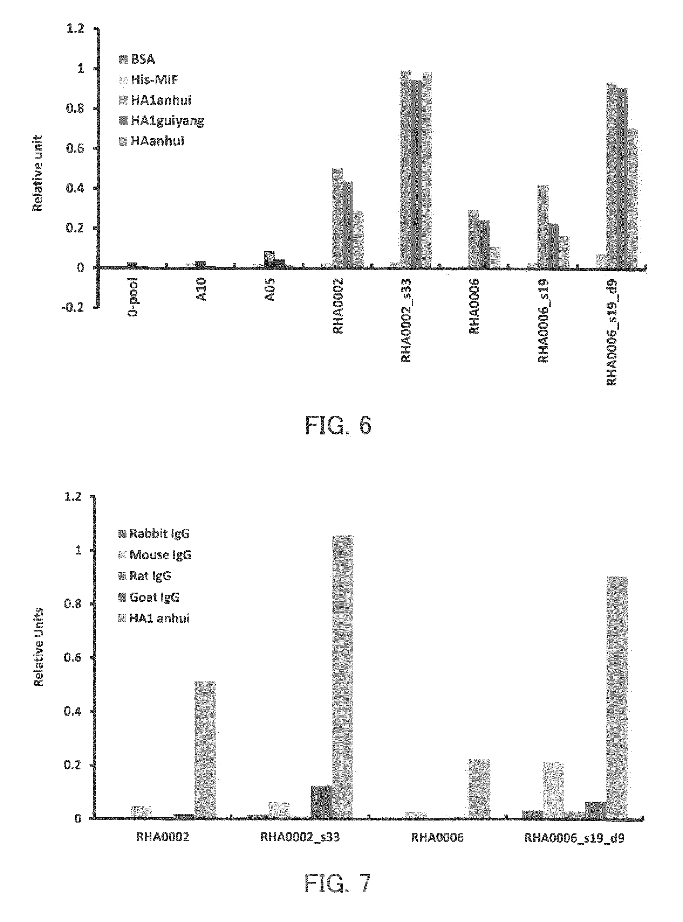

Except that the above aptamers were used, the binding ability to target proteins was analyzed in the same manner as in Example A1. The results thereof are shown in FIG. 6. FIG. 6 is a graph showing the binding ability of each aptamer to each target protein. In the graph of FIG 6, the vertical axis indicates the relative value of the signal intensity (RU) measured using the ProteON® XRP36. The relative value was determined in the same manner as in the item (3) in Example A1. In FIG. 6, "0-tool" indicates the result obtained regarding the N30.

As can be seen from FIG. 6, the aptamers of the present example all exhibited binding ability to HA1_{Anhui}, HA1_{Guiyang}, and HA_{Anhui}, whereas they did not bind to either BSA or His-MIF. In particular, RHA0002_s33 (SEQ ID NO: 17) and RHA0006_s19_d9 (SEQ ID NO: 32) exhibited high binding ability.

### (3) Cross-reaction

Cross-reactions of the above aptamers were examined in the same manner as in Example A1. The results thereof are shown in FIG. 7. FIG. 7 is a graph showing the binding ability of each aptamer to each antibody. In the graph of FIG. 7, the vertical axis indicates the relative value of the signal intensity (RU), as in FIG 6.

As can be seen from FIG. 7, the aptamers of the present example exhibited potent binding ability to the HA1_{Anhui}, whereas they did not exhibit binding ability to any of the antibodies. These results demonstrate that, for example, at the time of detecting HA using the aptamer of the present invention, an antibody also can be used in combination.

### [Example A5]

The present example examined the dissociation constant of each of aptamers RHA0002_s33 (SEQ ID NO: 17) and RHA0006_s19_d9 (SEQ ID NO: 32) against HA.

SPR was carried out in the same manner as in Example A1, except that the concentration of the biotinylated poly T was set to 100 nmol/L and the concentration of each of HA1_{Anhui}, HA1_{Guiyang}, and HA_{Anhui} was set to a predetermined value (15.6, 31.3, 62.5, 125, or 250 nmol/L). Then, from the results of the SPR, the dissociation constant of each aptamer was calculated. The results thereof are shown in Table 3 below.

**[Table 3]**

| RHA0002_s33 | | | | |
|---|---|---|---|---|
| | Kₐ(1/Ms) | K_{d}(1/s) | K_{d}(M) | Rmax (RU) |
| HA1_{Anhui} | 3.07E+04 | 5.30E-04 | 1.72E+08 | 4.75E+02 |
| HA1_{Guiyang} | 5.77E+04 | 1.31E-03 | 2.26E-08 | 2.35E+02 |
| HA_{Anhui} | 1.93E+04 | 5.16E-04 | 2.67E-08 | 1.04E+03 |
| | | | | |

| RHA0006_s19_d9 | | | | |
|---|---|---|---|---|
| | Kₐ (1/Ms) | K_{d}(1/s) | K_{D}(M) | Rmax (RU) |
| HA1_{Anhui} | 3.62E+04 | 5.54E-04 | 1.53E-08 | 3.70E+02 |
| HA1_{Guiyang} | 5.17E+04 | 1.07E-03 | 2.08E-08 | 3.61E+02 |
| HA_{Anhui} | 1.98E+04 | 4.90E-04 | 2.47E-08 | 9.81E+02 |

As can be seen from Table 3, RHA0002_s33 (SEQ ID NO: 17) and RHA0006_s19_d9 (SEQ ID NO: 32) each exhibited a very low dissociation constant against each target protein, which means that they have very high binding ability to each target protein.

### [Example A6]

The aptamer of the present invention, HA1 or HA, and an antibody were subjected to a binding reaction in this temporal order, and the binding ability thereof was analyzed by SPR.

The aptamers used in the present example were RHA0002_s33 (SEQ ID NO: 17) and RHA0006_s19_d9 (SEQ ID NO:32). HA1_{Anhui} was used as HA1, and HA_{Anhui} was used as HA. The antibodies used in the present example were Control Ab (rabbit immunoglobulin, Beckman Coulter, Inc., #731642) as a control antibody and Anti HA Ab (rabbit anti-HA antibody, Sino Biological Inc., #11048-RP02).

SPR was carried out in the same manner as in the item (3) in Example A1, except that the conditions of the SPR were set as follows. First, a ligand at 5000 nmol/L was injected to a flow cell of the sensor chip using ultrapure water (DDW), and the binding was allowed to proceed until the signal intensity (RU: Resonance Unit) reached about 1000 RU. As the ligand, biotinylated poly(dT) obtained by biotinylating the 5' end of 24-mer deoxythymidine was used. Then, the poly(dA)-added aptamer at 400 nmol/L was injected to the flow cell of the chip using an SPR buffer at a flow rate of 25 µL/min for 80 seconds, and the binding was allowed to proceed until the signal intensity reached about 700 RU. Next, using the SPR buffer, the target protein at 500 nmol/L was injected at a flow rate of 25 µL/min for 80 seconds and a detection antibody was then injected at 1 µmol/L at a flow rate of 50 µL/min for 120 seconds. Finally, washing was performed by flowing the SPR buffer.

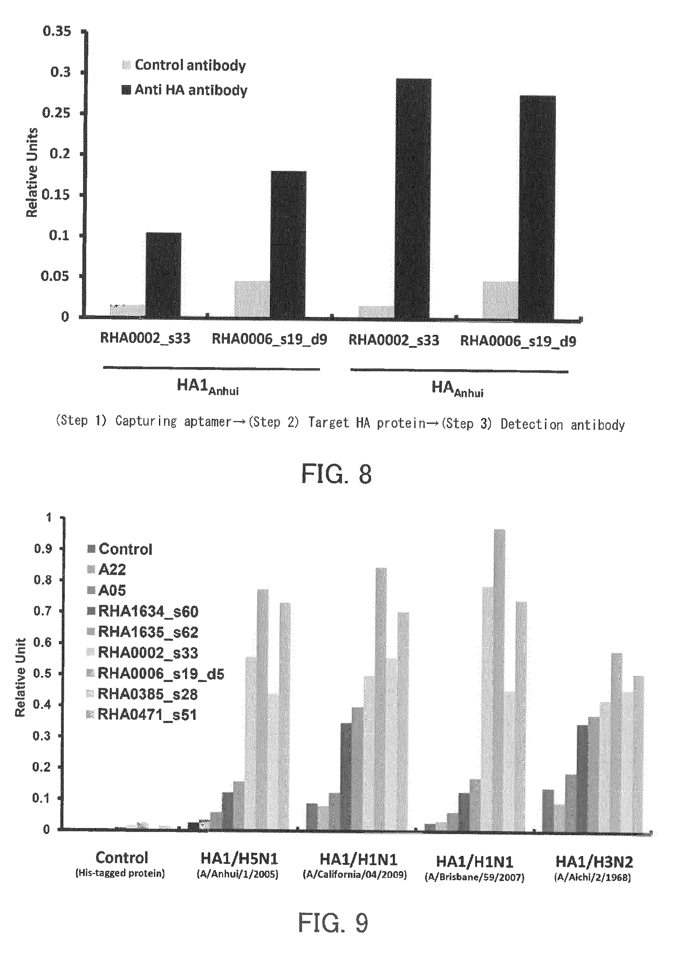

The results thereof are shown in FIG. 8. FIG. 8 is a graph showing the binding ability between each aptamer, each target protein, and each antibody. In the graph of FIG. 8, the vertical axis indicates the relative value of the signal intensity (RU) measured using the Prote ON® XRP36. The relative value was determined in the same manner as in the item (3) in Example A1.

As can be seen from FIG. 8, when either aptamer was used, the binding between the aptamer, HA or HA1, and the control antibody was not observed, and the binding between the aptamer, HA or HA1, and the anti-HA antibody was observed. These results demonstrate that the aptamer of the present invention can be used in combination with an antibody.

### [Example B1]

The present example examined the binding ability of each aptamer to HA by SPR.

### (1) Aptamers

As aptamers of the present example, the following polynucleotides were synthesized.
RHA0002_s33 (SEQ ID NO: 17)
GTGTTTGATGGTTTGGTCTGGTTGGCTTAACAC
RHA0006_s19_d5 (SEQ ID NO: 33)
GGGTTTGGGTTGGGTTGGGTTTTTGGGTTTGGGTTGGGTTGGG 
RHA1634_s60 (SEQ ID NO: 21)
GGGCACCCTCTCGCTGTACGGTTGATGCACGTTTCTTTCTCTTTATATTGCTGGGTG
CCC
 
RHA1635_s62 (SEQ ID NO: 23)
GGGGCCCACCCTCTCGCTGGCGGCTCTGTTCTGTTCTCGTCTCCTTGATTTCTGTGG
GCCCC
 RHA0385_s28 (SEQ ID NO: 25)
TTGGGGTTATTTTGGGAGGGCGGGGGTT
RHA0471_s51 (SEQ ID NO: 27)
TCCTCGTTGGGGGTGGTGGTGGGTTTCGGTTCATGGGTGGGACGGAGAGGA

A control nucleic acid molecule that does not exhibit binding properties to HA, an aptamer A22 of Non-Patent Document 1, which has been reported not to exhibit binding properties to HA, and the above-described aptamer A05 of Non-Patent Document 2 were synthesized.
Control (SEQ ID NO: 39)
CGGCGTTTGGTTGGCGTGAACCATTTTTAAGTT

Comparative Example A22 (SEQ ID NO: 40)

AATTAACCCTCACTAAAGGGCTGAGTCTCAAAACCGCAATACACTGGTTGTATGGT
CGAATAAGTTAA

Comparative Example A05 (SEQ ID NO: 53)
GAATTCAGTCGGACAGCGGGGGGAATTCTAGCAGTACACCTCGAGAATTATAGCC
AGGATGGACGAATATCGTCTCCC

To the 3' end of each of the above-described aptamers, 24-mer polydeoxyadenine (poly(dA)) was added. The thus-obtained poly(dA)-added aptamers were used in SPR to be described below.

### (2) Target proteins

Target proteins used in the present example were A/Anhui/1/2005-derived HA1 (HA1_{Anhui}: SEQ ID NO: 36) in Example A1, and A/California/04/2009-derived HA1, A/Brisbane/59/2007-derived HA1, and A/Aichi/2/1968-derived HA1, which are shown below.

### HA1_{California}

A/California/04/2009-derived HA1
Sino Biological Inc. #1055-V08H4

SEQ ID NO: 41
MKAILVVLLYTFATANADTLCIGYHANNSTDTVDTVLEKNVTVTHSVNLL
EDKHNGKLCKLRGVAPLHLGKCNIAGWILGNPECESLSTASSWSYIVETP
SSDNGTCYPGDFIDYEELREQLSSVSSFERFEIFPKTSSWPNHDSNKGVT
AACPHAGAKSFYKNLIWLVKKGNSYPKLSKSYINDKGKEVLVLWGIHHPS
TSADQQSLYQNADTYVFVGSSRYSKKFKPEIAIRPKVRDQEGRMNYYWTL
VEPGDKITFEATGNLVVPRYAFAMERNAGSGIIISDTPVHDCNTTCQTPK
GAINTSLPFQNIHPITIGKCPKYVKSTKLRLATGLRNIPSIQSR

### HA1_{Brisbane}

A/Brisbane/59/2007-derived HA1
Sino Biological Inc. #11052-V08H1

SEQ ID NO: 42
MKVKLLVLLCTFTATYADTICIGYHANNSTDTVDTVLEKNVTVTHSVNLL
ENSHNGKLCLLKGIAPLQLGNCSVAGWILGNPECELLISKESWSYIVEKP
NPENGTCYPGHFADYEELREQLSSVSSFERFEIFPKESSWPNHTVTGVSA
SCSHNGESSFYRNLLWLTGKNGLYPNLSKSYANNKEKEVLVLWGVHHPPN
IGDQKALYHTENAYVSVVSSHYSRKFTPEIAKRPKVRDQEGRINYYWTLL
EPGDTIIFEANGNLIAPRYAFALSRGFGSGIINSNAPMDKCDAKCQTPQG
AINSSLPFQNVHPVTIGECPKYVRSAKLRMVTGLRNIPSIQSR

### HA1_{Aichi}

A/Aichi/2/1968-derived HA1
Sino Biological Inc. #11707-V08H1

SEQ ID NO: 43
MKTIIALSYIFCLALGQDLPGNDNSTATLCLGHHAVPNGTLVKTITDDQI
EVTNATELVQSSSTGKICNNPHRILDGIDCTLIDALLGDPHCDVFQNETW
DLFVERSKAFSNCYPYDVPDYASLRSLVASSGTLEFITEGFTWTGVTQNG
GSNACKRGPGSGFFSRLNWLTKSGSTYPVLNVTMPNNDNFDKLYIWGIHH
PSTNQEQTSLYVQASGRVTVSTRRSQQTIIPNIGSRPWVRGLSSRISIYW
TIVKPGDVLVINSNGNLIAPRGYFKMRTGKSSIMRSDAPIDTCISECITP
NGSIPNDKPFQNVNKITYGACPKYVKQNTLKLATGMRNVPEKQTR

### (3) Analysis of binding ability by SPR

SPR was carried out in the same manner as in the item (3) in Example A1, except that the above aptamers and target proteins were used. The results thereof are shown in FIG. 9. FIG. 9 is a graph showing the binding ability of each aptamer to each target protein. In the graph of FIG. 9, the vertical axis indicates the relative value of the signal intensity (RU) measured using the ProteON® XRP36. The relative value was determined in the same manner as in the item (3) in Example A1.

As can be seen from FIG. 9, the aptamers according to the present example all exhibited higher binding ability to each HA1 than the aptamers according to the comparative examples.

### [Example B2]

Using an aptamer, immobilized HA was detected by ELAA (Enzyme-linked Aptamer Assay).

### (1) Aptamers

Aptamers used in the present example were RHA0006_s19_d5 (SEQ ID NO: 33), RHA1634_s60 (SEQ ID NO: 21), RHA1635_s62 (SEQ ID NO: 23), RHA0385_28 (SEQ ID NO: 25), and RHA0471_s51 (SEQ ID NO: 27), which were synthesized in Example B1. Also, the same control nucleic acid molecule and aptamers of the comparative examples as in Example B1 were used. The 3' end of each of these aptamers was biotinylated, and the thus-obtained biotinylated aptamers (detection aptamers) were used in ELAA to be described below.

### (2) Target proteins

Target proteins used in the present example were A/Anhui/1/2005-derived HA (HA/H5N1) and A/California/04/2009-derived HA (HA/H1N1), which were used in Examples A1 and B1, respectively.

### (3) ELAA (direct method)

The target was immobilized in wells, and the detection aptamer was brought into contact with the immobilization target. By detecting the detection aptamer bound to the immobilized target, the immobilized target was detected. This method hereinafter is referred to as a direct method of ELAA.

First, the HA was diluted with a carbonic acid buffer (pH 9.6), and 100 µL of the thus-obtained 10 µg/mL HA was added to each well of a 96-well plate (trade name: Immuno 96 Microwell Plates MaxiSorp: Nunc). The HA was allowed to adsorb to the wells at 4°C overnight. The wells were then washed with 200 µL of a washing buffer (50 mmol/L Tris, 100 mmol/L NaCl, 5 mmol/L KCl, 1 mmol/L MgCl2, 0.01 % Tween® 20, pH 7.4). Thereafter, 200 µL of a blocking buffer (Protein-Free (TBS) blocking buffer, #37570, PIERCE) was added, and the plate was incubated at room temperature for 1 hour. After the incubation, the wells were washed three timed with 200 µL of the washing buffer. Thus, the plate having the HA immobilized thereon was prepared.

Then, with a reaction solution (50 mmol/L Tris, 100 mmol/L NaCl, 5 mmol/L KCl, 1 mmol/L MgCl₂, 0.01% Tween® 20, 0.05% BSA, pH 7.4), the biotinylated aptamer was diluted to 1 µmol/L. Thereafter, 100 µL of the diluted biotinylated aptamer was added to each well, and the plate was incubated at room temperature for 1 hour. Subsequently, the wells were washed with the washing solution. Thereafter, 100 µL of 1000-fold diluted streptavidin-horseradish peroxidase (SA-HRP: GE, #RPN1231_2ML) was added, and the reaction was allowed to proceed at room temperature for 30 minutes. The wells were then washed with the washing solution. Thereafter, 100 µL of a TMB-E substrate (Moss Inc.) was added, and the reaction (color development) was allowed to proceed at room temperature for 20 minutes. Then, 100 µL of 0.5 mol/L sulfuric acid was added to the wells to terminate the reaction. Thereafter, the absorbance at a wavelength of 450 nm (reference wavelength: 620 nm) was measured using a plate reader.

As a blank, a 96-well plate without HA immobilized thereon was subjected to ELAA in the same manner as in the above.

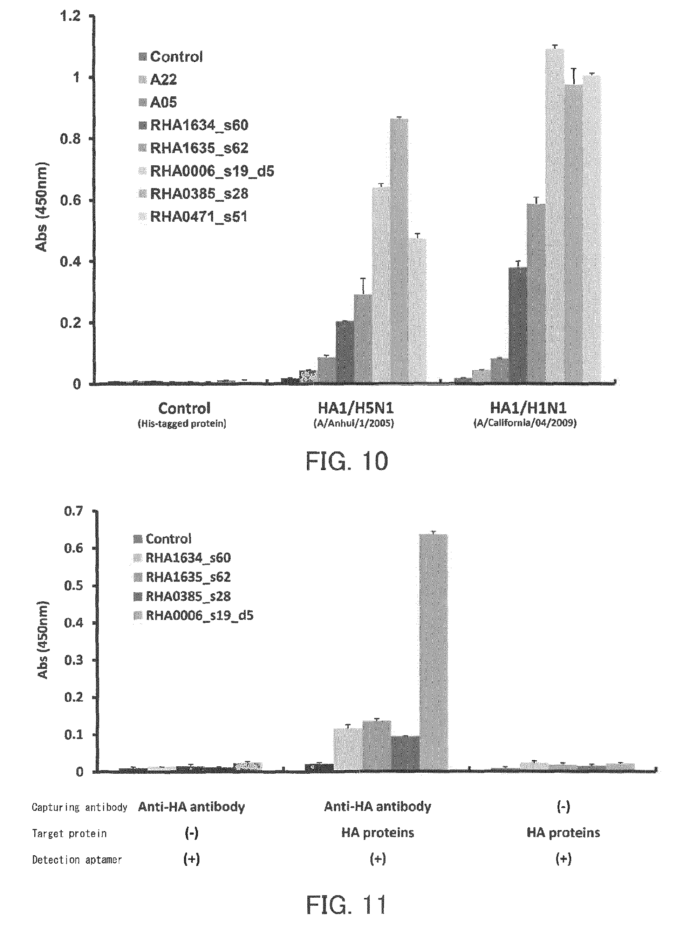

The results thereof are shown in FIG. 10. FIG 10 is a graph showing the amount of each HA bound to each aptamer. In FIG. 10, the vertical axis indicates the absorbance at a wavelength of 450 nm (reference wavelength: 620 nm), which indicates the binding ability.

As can be seen from FIG. 10, the aptamers according to the present example all exhibited higher binding ability to each HA than the aptamers according to the comparative examples. Regarding the blank, the absorbance was below the detection limit. From these results, it was found that the aptamers of the present example exhibited the binding properties not to the plate but to the immobilized HA1. According to such a method, the measured absorbance indicates the amount of an aptamer bound to immobilized HA, and hence, indirect qualification and quantification of the HA are possible. Moreover, because qualification and quantification of HA are possible, qualification and quantification of influenza virus also are possible.

### [Example B3]

Using an aptamer, HA captured by an immobilized antibody was detected by ELAA (Enzyme-linked Aptamer Assay).

### (1) Aptamers

Aptamers used in the present example were RHA0002_s33 (SEQ ID NO: 17), RHA0006_s19_d5 (SEQ ID NO: 33), RHA1634_s60 (SEQ ID NO: 21), and RHA1635_s62 (SEQ ID NO: 23), which were synthesized in Example B1. Also, the same control nucleic acid molecule as in Example B1 was used. The 3' end of each of these aptamers was biotinylated, and the thus-obtained biotinylated aptamers were used in ELAA to be described below.

### (2) Target protein

As a target protein, A/Anhui/1/2005-derived HA (HA1/H5N1) used in Example B1 was used.

### (3) Antibody

As a polyclonal antibody exhibiting binding properties to HA/H5N1, a rabbit anti-hemagglutinin (H5N1) pAb (Sino Biological Inc., #11048-RP2) was used.

### (4) ELAA

1 µg of the antibody was added to each well of a 96-well plate (trade name: Immuno 96 Microwell Plates MaxiSorp, Nunc). The plate was incubated at 4°C overnight to immobilize the antibody. As a blocking buffer, a Protein-Free (TBS) blocking buffer (trade name, #37570, PIERCE) was used, and the blocking treatment was carried out at room temperature for 1 hour. Then, 1 µg of the HA was added to each well of the plate. The plate was incubated at room temperature for 2 hours, thereby causing the HA to bind to the immobilized antibody on the plate. Subsequently, 100 µL of the biotinylated aptamer at 1 µmol/L was added to each well of the plate, and the plate was incubated at room temperature for 1 hour. Thereafter, a color-developing reaction was caused using streptavidin-HRP (×1000), and the absorbance was measured. Unless otherwise stated, the ELAA in the present example was carried out under the same conditions as those for the ELAA in Example B2.

As blanks, the absorbance measurement also was performed with respect to a system not containing the HA and a system in which the antibody had not been immobilized.

The results thereof are shown in FIG 11. FIG. 11 is a graph showing the amount of the HA bound to each aptamer. In FIG. 11, the vertical axis indicates the absorbance at a wavelength of 450 nm (reference wavelength: 620 nm), which indicates the binding ability.

As can be seen from FIG. 11, regarding each HA, the aptamers according to the present example all exhibited higher absorbances than the control nucleic acid molecule. In particular, RHA0006_s19_d5 (SEQ ID NO: 33) exhibited a very high absorbance. Regarding the blanks, in each of the system not containing the HA and the system in which the antibody had not been immobilized, the absorbance was below the detection limit. From these results, it was found that the aptamers of the present example exhibited binding properties not to the antibody or the plate but to the HA bound to the plate via the antibody. According to such a method (Sandwich-ELAA), the measured absorbance indicates the amount of an antibody bound to HA captured by an aptamer, and hence, indirect qualification and quantification of the HA are possible.

### [Example B4]

HA was captured by an immobilized aptamer, and the HA was detected using a primary antibody and a secondary antibody.

### (1) Aptamer

RHA0002_s33 (SEQ ID NO: 17) synthesized in Example B1 was used. Also, the same control nucleic acid molecule as in Example B1 was used. To the 3' end each of these aptamers, an amino group (-NH₂) was introduced. The thus-obtained aminated aptamers were used.

### (2) Target protein

As a target protein, A/Anhui/1/2005-derived HA (HA1/H5N1) used in Example B1 was used.

### (3) Antibodies

A primary antibody used in the present example was rabbit anti-hemagglutinin (H5N1) pAb (Sino Biological Inc., #11048-RP2), which is a polyclonal antibody exhibiting binding properties to HA/H5N1. A secondary antibody used in the present example was HRP-labeled anti-rabbit IgG antibody (α-Rabit IgG-HRP, #NA934-1ML, GE), which is a polyclonal antibody capable of binding to the primary antibody.

### (4) ELAA

To a 96-well plate (trade name: immobilized Amino plate, #436006, Nunc), the aminated aptamer diluted with a carbonic acid buffer (pH 9.6) was added. The plate was incubated at 4°C overnight to immobilize the aminated aptamer. The concentration of the aminated aptamer in each well of the plate was set to 0, 0.002, 0.02, 0.2, or 2 µM. The first blocking treatment was performed at room temperature for 1 hour using 1 mol/L Tris (pH 7.4). The second blocking treatment was performed at room temperature for 1 hour using a Protein-Free (TBS) blocking buffer (trade name, #37570, PIERCE) as a blocking buffer. Then, 1 µg of the Ha was added to each well of the plate. The plate was incubated at room temperature for 2 hours, thereby causing the HA to bind to the immobilized aptamer on the plate. Subsequently, 100 µL of the primary antibody at 100 nµM was added to each well of the plate, and the plate was incubated at room temperature for 1 hour. Thereafter, the plate was washed. The secondary antibody (×5000) further was added thereto, and the plate was incubated at room temperature for 30 minutes. Thereafter, a color-developing reaction was caused using a TMB-E substrate, and the absorbance was measured. Unless otherwise stated, the ELAA in the present example was carried out under the same conditions as those for the ELAA in Example B2.

As a blank, the absorbance measurement also was performed with respect to a system in which, instead of the primary antibody, rabbit immunoglobulin (Beckman Coulter, Inc., #731642) was used as a control antibody.

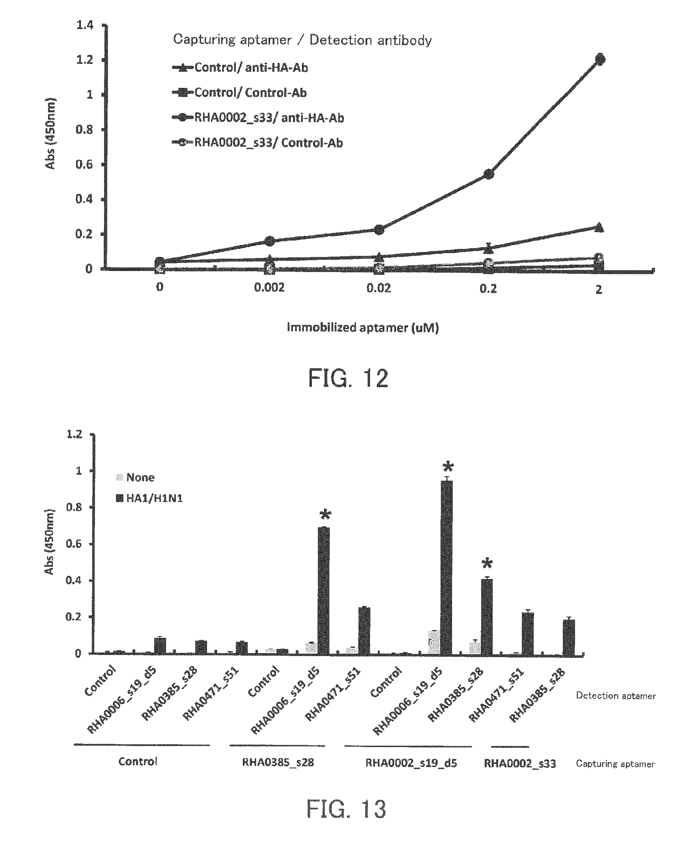

The results thereof are shown in FIG. 12. FIG. 12 is a graph showing the amount of the HA bound to the aptamer. In FIG. 12, the vertical axis indicates the absorbance at a wavelength of 450 nm (reference wavelength: 620 nm), which indicates the binding ability.

As can be seen from FIG. 12, the absorbance increased in a manner dependent on the concentration of the immobilized RHA0002_s33 (SEQ ID NO: 17) immobilized on the plate. From this result, it was found that the aptamer of the present example can capture HA in a concentration-dependent manner. According to such a method (Sandwich-ELAA), the measured absorbance indicates the amount of an antibody bound to captured HA, and hence, indirect qualification and quantification of the HA are possible.

### [Example B5]

HA was captured by an immobilized capturing aptamer, and the HA was detected using a detection aptamer.

### (1) Aptamers

Aptamers used in the present example were RHA0002_s33 (SEQ ID NO: 17), RHA0006_s19_d5 (SEQ ID NO: 33), RHA0385_s28 (SEQ ID NO: 25), and RHA0471_s51 (SEQ ID NO: 27), which were synthesized in Example B1. The capturing aptamer to be immobilized on a plate was configured so that an amino group (-NH₂) was introduced to the 3' end thereof. The detection aptamer was configured so that the 3' end thereof was biotinylated. Also, the same control nucleic acid molecule as in Example B1 was used. The combinations of the capturing aptamer and the detection aptamer are shown below.

**[Table 4]**

| Capturing aptamer | Detection aptamer |
|---|---|
| Control | RHA0006_s19_d5 |
| | RHA0385_s28 |
| | RHA0471_s51 |
| RHA0385_s28 | Control |
| | RHA0006_s19_d5 |
| | RHA0471_s51 |
| RHA0006_s19_d5 | Control |
| | RHA0006_s19_d5 |
| | RHA0385_s28 |
| | RHA0471_s51 |
| RHA0002_s33 | RHA0385_s28 |

### (2) Target protein

As a target protein, A/California/04/2009-derived HA (HA1/H1N1) used in Example B1 was used.

### (3) ELAA (sandwich method)

The capturing aptamer was immobilized on each well, and a target was brought into contact with the capturing aptamer. Further, the detection aptamer was brought into contact with the target bound to the capturing aptamer, thereby sandwiching the target between the capturing aptamer and the detection aptamer. Then, the target was detected by detecting the detection aptamer in this complex. This method hereinafter is referred to as a sandwich method of ELAA.

First, 100 µL of the capturing aptamer at 0.2 µmol/L was added to each well of a 96-well plate (trade name: immobilized Amino plate, #436006, Nunc). The plate was then was incubated at room temperature for 2 hours to immobilize the aminated aptamer. The first blocking treatment was performed at room temperature for 1 hour using 1 mol/L Tris (pH 7.4). The second blocking treatment was performed at room temperature for 1 hour using a Protein-Free (TBS) blocking buffer (trade name, #37570, PIERCE) as a blocking buffer. Then, 1 µg of the Ha was added to each well of the plate. The plate was incubated at room temperature for 2 hours, thereby causing the HA to bind to the capturing aptamer on the plate. Subsequently, 100 µL of the detection aptamer at 1 µmol/L was added to each well of the plate, and the plate was incubated at room temperature for 1 hour. Thereafter, a color-developing reaction was caused using streptavidin-HRP (×1000), and the absorbance was measured. Unless otherwise stated, the ELAA in the present example was carried out under the same conditions as those for the ELAA in Example B2.

The results thereof are shown in FIG. 13. FIG 13 is a graph showing the amount of the HA bound to the detection aptamer bound to the HA captured by the capturing aptamer.

As can be seen from FIG. 13, when the aptamers of the present example were used as the capturing aptamer and the detection aptamer, higher absorbances were exhibited as compared with the case where the control was used. In particular, very high absorbances were exhibited when the combination of the capturing aptamer and the detection aptamer was as follows: the combination of RHA0385_s28 (SEQ ID NO: 25) and RHA0006_s19_d5 (SEQ ID NO: 33); and the combination of RHA0006_s19_d5 (SEQ ID NO: 33) and RHA0006_s19_d5 (SEQ ID NO: 33) or RHA0385_s28 (SEQ ID NO: 25). According to such a method (Sandwich-ELAA), the measured absorbance indicates the amount of the detection aptamer bound to the HA captured by the capturing aptamer, and hence, indirect qualification and quantification of the HA are possible.

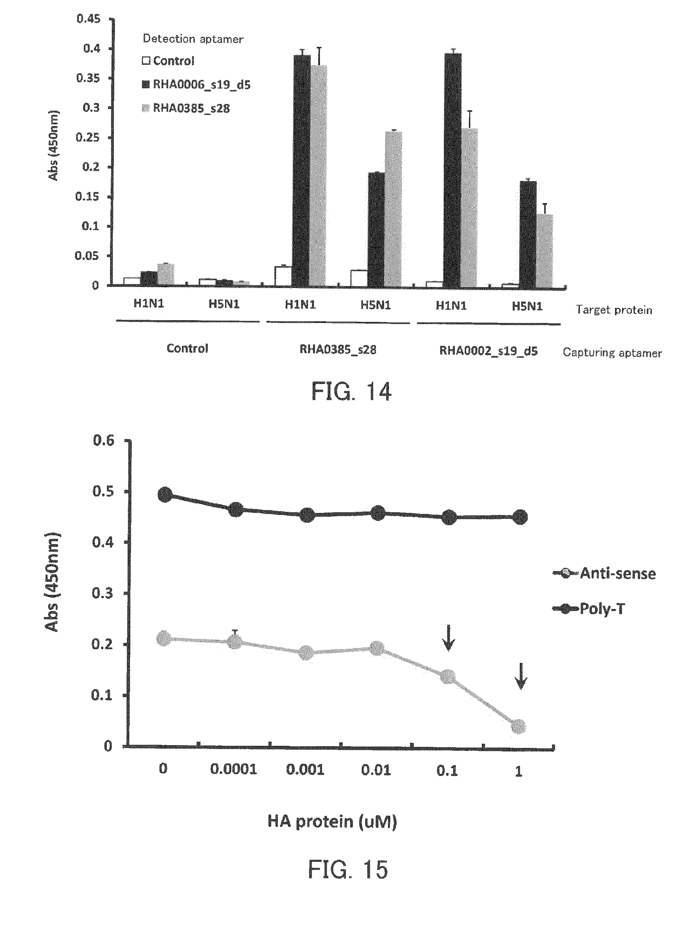

ELLA was carried out in the same manner as described above, except that: A/Anhui/1/2005-derived HA (HA_{Anhui}) also was used as a target protein in addition to the A/California/04/2009-derived HA (HA_{California}); and the combination of RHA0006_s19_d5 (SEQ ID NO: 33) and RHA0385_s28 (SEQ ID NO: 25) was employed as the combination of the capturing aptamer and the detection aptamer. The results thereof are shown in FIG. 14. FIG 14 is a graph showing the amount of the HA bound to the detection aptamer bound to the HA captured by the capturing aptamer. As can be seen from FIG. 14, high absorbances were observed not only when the target protein was HA_{California} but also when the target protein was HA_{Anhui}. From these results, it was found that, by using a capturing aptamer and a detection aptamer, indirect qualification and quantification of HA are possible.

### [Example B6]

### (1) Aptamer

RHA0006_s19_d5 (SEQ ID NO: 33) synthesized in Example B1 was used. 24-mer polydeoxyadenine (poly(dA)) was added to the 3' end of the aptamer, and further, the 3' end of the poly(dA) was biotinylated. The thus-obtained biotinylated poly(dA)-added aptamer was used.

### (2) Antisense and poly(dT)

An antisense (SEQ ID NO: 44) having a sequence complementary to the aptamer was synthesized, and the 5' end thereof was aminated.
Antisense (SEQ ID NO: 44)
CCCAAACCCAACCCAACCCAAAAACCCAAACCCAACCCAACCCTTTTT

### (3) Target protein

As a target protein, A/California/04/2009-derived HA (HA/H5N1) used in Example B1 was used.

### (4) ELAA

100 µL of the aminated antisense at 0.02 µmol/L was added to each well of a 96-well plate (trade name: immobilized Amino plate, #436006, Nunc). The plate was then incubated at room temperature for 2 hours to immobilize the aminated antisense. The first blocking treatment was performed at room temperature for 1 hour using 1 mol/L Tris (pH 7.4). The second blocking treatment was performed at room temperature for 1 hour using a Protein-Free (TBS) blocking buffer (trade name, #37570, PIERCE) as a blocking buffer.

On the other hand, the HA at a predetermined concentration (0, 0.0002, 0.002, 0.02, 0.2, or 2 µmol/L) was mixed with the biotinylated poly(dA)-added aptamer at 2 nmol/L, and the resultant mixture was incubated at 4°C overnight, thereby causing the binding between the HA and the aptamer. Then, this mixture was added to the well to achieve a concentration of 1 nmol/L. The plate was incubated at room temperature for 1 hour, and then washed. Thereafter, a color-developing reaction was caused by streptavidin (SA)-HRP (×1000), and the absorbance was measured. Unless otherwise stated, the ELAA in the present example was carried out under the same conditions as those for the ELAA in Example B2.

As a comparative example, the absorbance measurement was performed in the same manner, except that, instead of the aminated antisense, 24-mer poly(dT) with the aminated 5' end was used.

The results thereof are shown in FIG. 15. FIG. 15 is a graph showing the amount of the aptamer bound to the immobilized antisense. In FIG. 15, the vertical axis indicates the absorbance at a wavelength of 450 nm (reference wavelength: 620 nm), which indicates the binding ability.

As can be seen from FIG. 15, when the antisense and the aptamer were used in combination, the absorbance decreases in correlation with the increase in the concentration of the HA. The reason for this is as follows. When the HA is not present, the aptamer can bind to the antisense immobilized on the plate. Thus, biotin contained in the aptamer captured on the plate via the antisense binds to the SA-HRP to cause color development. However, when the HA is present, the aptamer binds to the HA and thus cannot bind to the antisense immobilized on the plate. Accordingly, the aptamer is not captured on the plate. Thus, a color-developing reaction by SA-HRP does not occur. Therefore, the absorbance decreases in correlation with the increase in the concentration of the HA. On the other hand, when the poly(dT) is used, the poly(dT) can bind to the poly(dA) added to the aptamer. Thus, the decrease in absorbance accompanying the increase in HA was not observed. According to such a method (Competitive-ELAA), the measured absorbance indicates the amount of the aptamer that is captured by the antisense and is not bound to HA, and hence, indirect qualification and quantification of HA are possible.

### [Example B7]

The present example examined a cross-reaction of an aptamer with an antibody. Unless otherwise stated, the examination was carried out in the same manner as in the item (4) in Example A1.

As target proteins, A/Anhui/1/2005-derived HA1 (HA1_{Anhui}), BSA, and His-MIF were used instead of the A/California/04/2009-derived HA1 (HA1_{California}). Antibodies used in the present example were Rabbit IgG, Mouse IgG, Chicken IgG, Rat IgG, and Goat IgG described in the item (4) in Example A1.

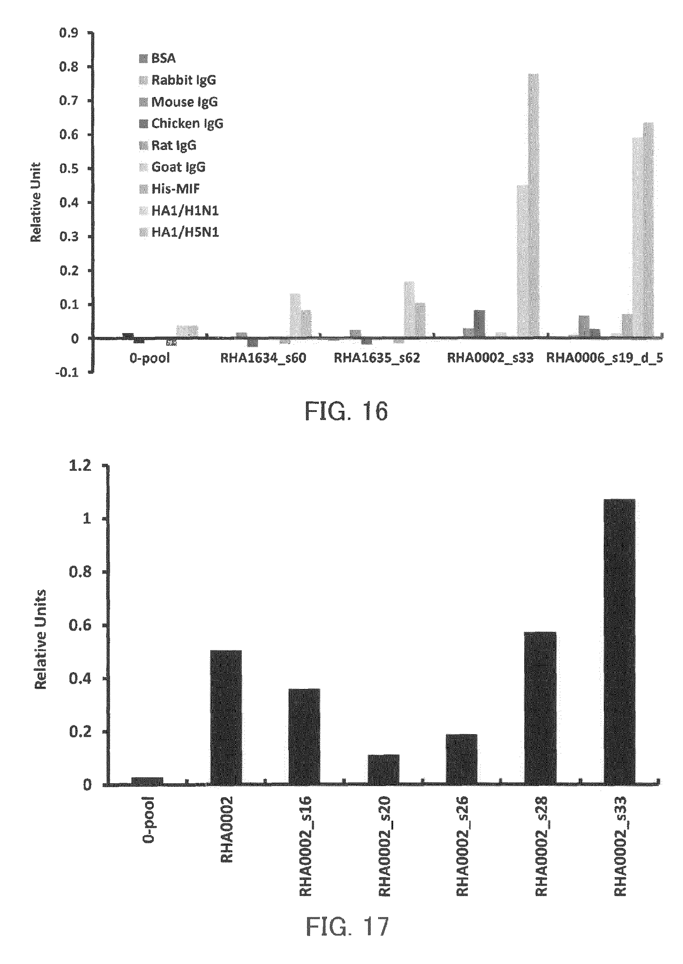

The results thereof are shown in FIG. 16. FIG. 16 is a graph showing the binding ability of each aptamer to each antibody. In the graph of FIG. 16, the vertical axis indicates the relative value of the signal intensity (RU), as in FIG. 4. The nine kinds of bars shown for each aptamer indicate, from the left, the results obtained regarding BSA, Rabbit IgG, Mouse IgG, Chicken IgG, Rat IgG, Goat IgG. His-MIF, HA1/H1N1, and HA1/H5N1.

As can be seen from FIG. 16, the aptamers of the present example exhibited potent binding ability to HA, whereas they exhibited little binding ability to any of the antibodies. These results demonstrate that, for example, at the time of detecting an influenza virus or HA using the aptamer of the present invention, an antibody also can be used in combination.

### [Example C1]

The present example examined the binding ability of each of the following aptamers to a target protein. Unless otherwise stated, the examination was carried out in the same manner as in the item (3) in Example A1.

### (1) Aptamers

RHA0002 (SEQ ID NO: 12)
GGTTAGCCCGTCCCGAGATAACTCGGGGTGTTTGATGGTTTGGTCTGGTTGGCTTA
ACACACGGCGGCTGTAG

RHA0002_s16 (SEQ ID NO: 13)
GGTTTGGTCTGGTTGG
RHA0002_s20 (SEQ ID NO: 14)
GTGGTTTGGTCTGGTTGGAC
RHA0002_s26 (SEQ ID NO: 15)
CGTTTGGTTTGGTCTGGTTGGCAACG
RHA0002_s28 (SEQ ID NO: 16)
CGTTATGGTTTGGTCTGGTTGGCTAACG
RHA0002_s33 (SEQ ID NO: 17)
GTGTTTGATGGTTTGGTCTGGTTGGCTTAACAC

### (2) Target protein

As the target protein, the HA1_{Anhui} was used.

The results thereof are shown in FIG. 17. FIG. 17 is a graph showing the binding ability of each aptamer to the target protein. In the graph of FIG. 17, the vertical axis indicates the relative value of the signal intensity (RU). The relative value was determined in the same manner as in the item (3) in Example A1. In FIG. 17, "0-pool" indicates the result obtained regarding the N30. As can be seen from FIG 17, the aptamers of the present example all exhibited binding ability to HA1_{Anhui}.

### [Example C2]

The present example examined the binding ability of each of the following aptamers to a target protein and an antibody. Unless otherwise stated, the examination was carried out in the same manner as in the item (4) in Example A1.

### (1) Aptamers

RHA0002_s33 (SEQ ID NO: 17)
GTGTTTGATGGTTTGGTCTGGTTGGCTTAACAC 
RHA0002_s33_d9 (SEQ ID NO: 48)
GTGTTTGATGGTTTGGTCTGGTTGGCTTAACACTTTTTTTTTGTGTTTGATGGTTTG
GTCTGGTTGGCTTAACAC
 RHA0006_s19 (SEQ ID NO: 19)
GGGTTTGGGTTGGGTTGGG
RHA0006_s19_d9 (SEQ ID NO: 32)
GGGTTTGGGTTGGGTTGGGTTTTTTTTTGGGTTTGGGTTGGGTTGGG
RHA0006_s19_d9_AT(SEQ ID NO: 46)
GGGTTTGGGTTGGGTTGGGATATATATAGGGTTTGGGTTGGGTTGGG
RHA0006_s19_d5 (SEQ ID NO: 33)
GGGTTTGGGTTGGGTTGGGTTTTTGGGTTTGGGTTGGGTTGGG

RHA0006_s19_d_R20 (SEQ ID NO: 47)
GGGTTTGGGTTGGGTTGGGGATTGGAATTAAGGCGTGTGGGGTTTGGGTTGGGTT
GGG

RHA0006_s19_t9 (SEQ ID NO: 51)

GGGTTTGGGTTGGGTTGGGTTTTTTTTTGGGTTTGGGTTGGGTTGGGTTTTTTTTTG

GGTTTGGGTTGGGTTGGG

### (2) Target proteins

As the target proteins, the HA1_{Anhui} and the HA_{Anhui} were used. As the antibody, Rabbit IgG described in Example A1 (4) was used.

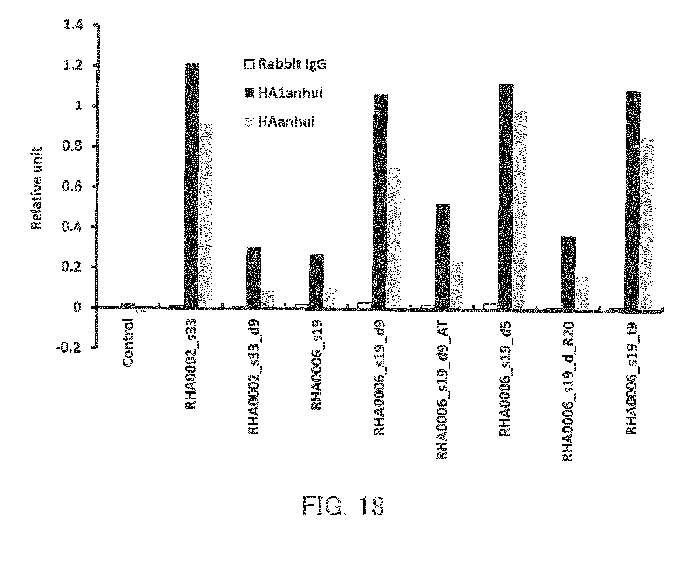

The results thereof are shown in FIG 18. FIG. 18 is a graph showing the binding ability of each aptamer to the respective target proteins and the antibody. In the graph of FIG 18, the vertical axis indicates the relative value of the signal intensity (RU). The relative value was determined in the same manner as in the item (3) in Example A1. In FIG 18, the "Control" indicates the result obtained regarding the N30.

As can be seen from FIG. 18, the aptamers of the present example all exhibited binding ability to HA1_{Anhui} and HA_{Anhui}, whereas they exhibited little binding ability to the antibody. Also, as a result of changing the length of the linker in the aptamer, RHA0006_s19_d5 (SEQ ID NO: 33) in which the linker length was 5-mer exhibited superior binding ability to RHA0006_s19_d9 (SEQ ID NO: 32) in which the linker length was 9-mer.

### [Example D1]

Using an aptamer, an immobilized influenza virus was detected by ELAA.

### (1) Viruses

Influenza viruses used in the present example are shown below. As a control virus, the following non-influenza virus was used.

### (Influenza viruses)

### H1N1

A/Brisbane/10/07
A/California/04/09
A/Georgia/20/06

### H3N2

A/Wisconsin/67/05
A/Brisbane/59/07
A/Moscow/10/99

### H5N1

A/Japanese white eye/Hong Kong/38/06

### (Non-influenza virus)

Sindbis virus

### (2) Virus stock preparation 1

Virus stocks of the H1N1, the H3N2, and the non-influenza viruses were prepared in the following manner.

An MDCK (Madin Darby Canine Kidney) cell line (ATCC® CCL-34 (trademark)) established from a dog's kidney was used to provide cells for viral research. A growth medium had the following composition (final concentration). Fetal bovine serum (FBS) as one of the components of the growth medium was DFBS (trade name, Mediatech), and the remaining components were products commercially available from Invitrogen. The culture conditions were set as follows: 37°C ± 2°C, with humidity, 5% CO₂, overnight. The culture plate was examined before use, and it was confirmed that the culture density was 80%.

### (Growth medium)

1 × minimum essential medium (MEM)
5% FBS
2mmol/L L-glutamine
3% NaHCO₃
1 × MEM Vitamins Solution

The MCDK cells confluent in a flask (about 9.6 × 10⁶ cells/mL) were infected with each type of virus. The cells were cultured using an infection medium having the following composition (final concentration). As the components of the infection medium, products commercially available from Life Technologies were used, unless otherwise stated. For the A/Brisbane/59/07 and the A/California/04/09, the multiplicity of infection (MOI) was set to 0.01 PFU/cell. For the A/Brisbane/10107, the A/Wisconsin/67/05, the A/Moscow/10/99, the A/Georgia/20/06, and the Sinbis, the multiplicity of infection (MOI) was set to 0.05 PFU/cell. PFU is a plaque formation unit.

### (Infection medium)

1 × MEM
3% NaHCO₃
1 × MEM Vitamins solution (Sigma)
1 × L-glutamine
100 U/mL penicillin
100 µg/mL streptomycin
50 µg/mL gentamicin

The cells were infected with the virus for 1 hour. Thereafter, modified trypsin was added to the flask to achieve a concentration of 2 µg/mL. As the modified trypsin, trypsin treated with L-(tosylamide-2-phenyl)ethyl chloromethyl ketone (TPCK) (trade name, TPCK treated trypsin, USB) was used.

Then, when the cytopathic effect (CPE) reached about 100%, the culture supernatant containing the virus was collected by centrifugation (200 × G, 10 minutes, 4°C). The remaining supernatant was subjected to centrifugation using ultrafilter membranes to remove proteins of 100 kDa or less and to concentrate the virus. As the ultrafilter membranes, Amicon Ultra centrifugal filter devices 100,000 NMWL (trade name, Millipore) were used, and the conditions for the centrifugation were 3000 × G, 4°C, and 30 minutes. This operation was repeated until the entire supernatant was concentrated. The thus-obtained concentrate was used as a semipurified virus stock. Part of the virus stock was collected. The virus contained therein was inactivated by UV irradiation, and the concentration of the virus was determined using a BCA assay (trade name, Pierce). The virus stock was stored at or below -65°C.

### (3) Virus stock preparation 2

A virus stock of the H5N1, which is an avian influenza virus, was prepared in the following manner.

Attenuated rg A/Japanese White Eye/Hong Kong/38/06 was inoculated into specific pathogen-free (SPF) hen eggs. The hen eggs were grown and infected with the virus. Then, 48 hours after the infection, a chorioallantoic fluid fraction containing the virus was collected. The collected fraction was then diluted with a diluent. As the diluent, 1 × PBS (pH 7.4) containing 100 U/mL penicillin, 100 µg/mL streptomycin, and 50 µg/mL gentamicin was used. The diluted fraction was used as a virus stock, without being concentrated as described in the item (2) above. The virus stock was stored at or below -65°C.

Because the H5N1 was amplified in the hen eggs as described above, it contained a large amount of hen egg-derived proteins. Thus, the purification step using a column described in the item "(2) Virus stock preparation 1" above was not performed. On this account, regarding the virus stock of the H5N1, the amount thereof to be used was indicated as the protein concentration, instead of the virus concentration. The H5N1 was used in such a manner that the protein concentration thereof would be 100 times greater (100×V µg/well) than the virus concentration (V µg/well) of the viruses other than the H5N1, in order to set the number of immobilized virus particles of the H5N1 to be comparable to those of the other viruses.

### (4) ELAA (direct method)

Aptamers used in the present example were: RHA0006_s19 (SEQ ID NO: 19) shown in Example A3; and RHA1635_s62 (SEQ ID NO: 23), RHA0385_s28 (SEQ ID NO: 25), and RHA0006_s19_d5 (SEQ ID NO: 33) shown in Example B1. Then, ELAA was carried out in the same manner as in Example B2, except that the virus was immobilized instead of the target protein. Furthermore, as a control, the same control nucleic acid molecule (SEQ ID NO: 39) as in Example B1 was used.

### (5) Binding ability to viruses

The binding ability of each aptamer to viruses was examined, and the results of the examination are shown below.

### (5-1)H5N1

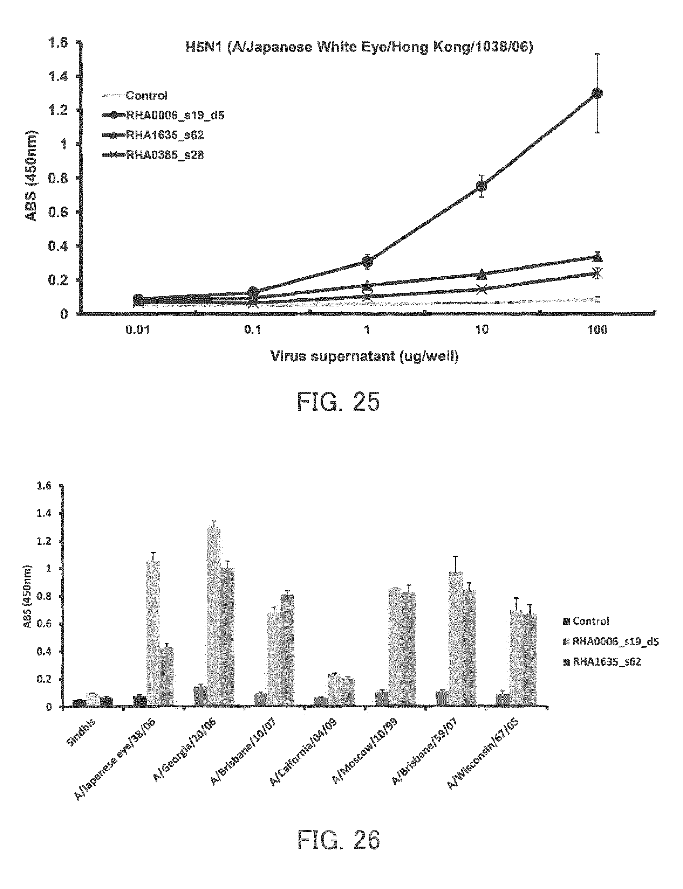

Regarding the RHA1635_s62 (SEQ ID NO: 23), the RHA0006_s19_d5 (SEQ ID NO: 33), and the RHA0385_s28 (SEQ ID NO: 25), their binding ability to A/Japanese white eye/Hong Kong/38/06 as H5N1 was examined. The concentration of each aptamer was set to 0.2 µmol/L. The results thereof are shown in FIG. 25. FIG 25 is a graph showing the amount of each aptamer bound to the virus. In FIG. 25, the vertical axis indicates the absorbance at a wavelength of 450 nm (reference wavelength: 620 nm), which indicates the binding ability, and the horizontal axis indicates the amount of the protein (µg/well) corresponding to the amount of the immobilized virus.

As can be seen from FIG. 25, the control nucleic acid molecule exhibited little binding properties to the influenza virus H5N1. In contrast, each of the aptamers exhibited high binding properties to the influenza virus H5N1.

Regarding RHA0006_s19_d5, the absorbance (not shown) indicating the binding properties to H1N1 (virus: 1 µg/well) was comparable to the absorbance indicating the binding properties to the H5N1 (protein: 100 µg/well) shown in FIG 25. On the basis of this result, in the following experiments, the H5N1 was immobilized so that the protein concentration thereof would be 100 times greater (100×V µg/well) than the virus concentration (V µg/well) of the viruses other than the H5N1, as described above.

### (5-2) H1N1, H3N2, and H5N1

The binding ability of the RHA1635_s62 (SEQ ID NO: 23) and the RHA0006_s19_d5 (SEQ ID NO: 33) to the influenza viruses H1N1, H3N2, and H5N1 and to Sindbis virus as the non-influenza virus were examined. The concentration of each aptamer was set to 0.2 µmol/L. For the viruses other than the H5N1, the amount of the immobilized virus was set to 1 µg/well. For the H5N1, instead of the amount of the immobilized virus, the amount of the protein corresponding thereto was set to 100 µg/well. The results thereof are shown in FIG. 26. FIG 26 is a graph showing the amount of each aptamer bound to each type of virus. In FIG. 26, the vertical axis indicates the absorbance at a wavelength of 450 nm (reference wavelength: 620 nm), which indicates the binding ability.

As can be seen from FIG. 26, the control nucleic acid molecule exhibited little binding properties to both the non-influenza virus and the influenza viruses. In contrast, each of the aptamers exhibited no binding properties to the non-influenza virus and exhibited high binding properties only to the influenza viruses. From these results, it was found that the aptamer of the present invention also is applicable to a direct method in influenza virus detection.

### [Example D2]

An influenza virus was captured by an immobilized capturing aptamer, and then the influenza virus was detected by a detection aptamer.

### (1) Viruses

Viruses used in the present example were the following viruses provided in Example D1.

### (Influenza viruses)

### H1N1

A/California/04/09
A/Georgia/20/06

### H3N2

A/Moscow/10/99

### H5N1

A/Japanese white eye/Hong Kong/38/06

### (non-influenza viruses)

Sindbis virus

### (2) ELAA (sandwich method)

As capturing aptamers, RHA0385_s28 (SEQ ID NO: 25) and RHA0006_s19_d5 (SEQ ID NO: 33) were used. As a biotinylated detection aptamer, RHA0006_s19_d5 (SEQ ID NO: 33) was used. ELAA was carried out in the same manner as in Example B3, except that: the virus was used instead of the target protein; for the H5N1, the amount of the protein was set to 1 µg/well; for the viruses other than the H5N1, the amount of the virus was set to 1 µg/well; the concentration of the capturing aptamer was set to 0.1 µmol/L; and the concentration of the biotinylated aptamer was set to 0.1 µmol/L. Furthermore, as a blank, ELAA was carried out in the same manner as in the above with respect to a system containing a buffer instead of the influenza virus. Also, as a control, ELAA was carried out in the same manner as in the above with respect to a system in which, instead of the aptamers, the control nucleic acid molecules (SEQ ID NO: 39) used in Example B1 were used.

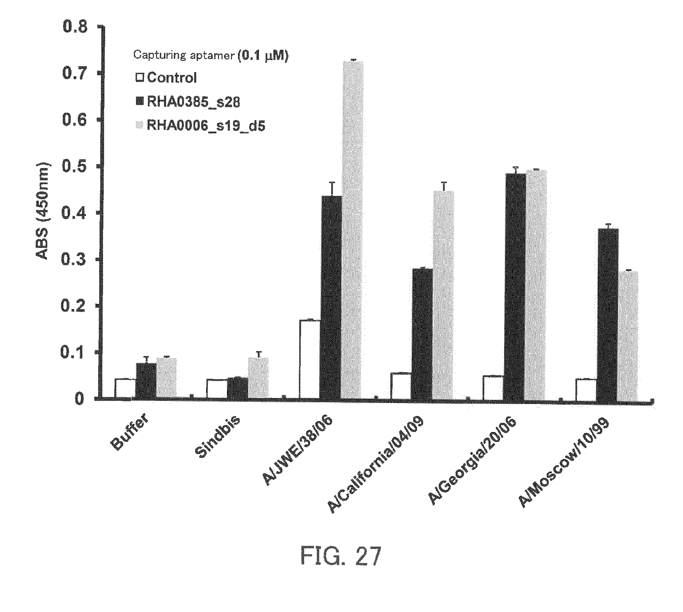

The results thereof are shown in FIG. 27. FIG 27 is a graph showing the amount of each influenza virus bound to the aptamer. In FIG. 27, the vertical axis indicates the absorbance at a wavelength of 450 nm (reference wavelength: 620 nm), which indicates the binding ability.

As can be seen from FIG. 27, the control nucleic acid molecule exhibited little binding properties to both the non-influenza virus and the influenza viruses. In contrast, each of the aptamers exhibited no binding properties to the non-influenza virus and exhibited high binding properties only to the influenza viruses. From these results, it was found that the aptamer of the present invention also is applicable to a sandwich method in influenza virus detection.

While the present invention has been described above with reference to embodiments and examples, the present invention is by no means limited thereto. Various changes and modifications that may become apparent to those skilled in the art may be made in the configuration and specifics of the present invention without departing from the scope of the present invention.

This application claims priority from Japanese Patent Application No. 2012-126861 filed on June 4, 2012. The entire disclosure of this Japanese patent application is incorporated herein by reference.

### Industrial Applicability

The nucleic acid molecule of the present invention can bind to influenza viruses, and thus can detect influenza viruses, for example. Therefore, the nucleic acid molecule of the present invention can be a very useful tool for the detection of influenza viruses in the fields of clinical practice, animal husbandry, and the like, for example.

## Claims

1. A nucleic acid molecule that binds to an influenza virus, the nucleic acid molecule comprising at least one polynucleotide selected from the group consisting of the following polynucleotides (a) to (d):
(a) a polynucleotide that has any of base sequences of SEQ ID NOs: 1 to 30;
(b) a polynucleotide that has a base sequence obtained by deletion, substitution, insertion, and/or addition of one or more bases in any of the base sequences of the polynucleotide (a) and binds to the influenza virus;
(c) a polynucleotide that has a base sequence with an identity of at least 80% to any of the base sequences of the polynucleotide (a) and binds to the influenza virus; and
(d) a polynucleotide that has a base sequence complementary to a polynucleotide hybridizing to any of the base sequences of the polynucleotide (a) under stringent conditions and binds to the influenza virus.

2. The nucleic acid molecule according to claim 1, comprising two or more polynucleotides selected from the polynucleotides (a) to (d).

3. The nucleic acid molecule according to claim 2, wherein the two or more polynucleotides are linked to each other via a linker.

4. The nucleic acid molecule according to claim 2 or 3, comprising two polynucleotides (RHA0006_s19) each having a base sequence of SEQ ID NO: 19 or two polynucleotides (RHA0002_s33) each having a base sequence of SEQ ID NO: 17, with the two polynucleotides being linked to each other via a linker.

5. The nucleic acid molecule according to claim 4, wherein the two polynucleotides linked to each other via the linker as a whole have a base sequence of SEQ ID NO: 31 or 45.

6. The nucleic acid molecule according to any one of claims 1 to 5, wherein the polynucleotide is DNA.

7. A binding agent that binds to an influenza virus, the binding agent comprising the nucleic acid molecule according to any one of claims 1 to 6.
